# EUROPÄISCHE PATENTSCHRIFT

(11) **EP 3 201 959 B1**
(45) Veröffentlichungstag und Bekanntmachung des Hinweises auf die Patenterteilung: **24.07.2019**
(21) Anmeldenummer: 14825342.0
(22) Anmeldetag: 22.12.2014
(51) Int. Cl.: H01L 51/54

(54) **ORGANISCHES ELEKTRONISCHES BAUTEIL**
ORGANIC ELECTRONIC COMPONENT
COMPOSANT ÉLECTRONIQUE ORGANIQUE

(30) Priorität: 30.09.2014 DE 102014114231
(43) Veröffentlichungstag der Anmeldung: 09.08.2017
(73) Patentinhaber: OSRAM OLED GmbH, 93049 Regensburg (DE)
(72) Erfinder: SCHMID, Günter, 91334 Hemhofen (DE); MALTENBERGER, Anna, 91359 Leutenbach (DE); PECQUEUR, Sébastien, 62136 La Couture (FR); REGENSBURGER, Stefan, 92318 Neumarkt (DE)
(74) Vertreter: Epping - Hermann - Fischer
(86) Internationale Anmeldenummer: PCT/EP2014/079049
(87) Internationale Veröffentlichungsnummer: WO 2016/050330

(56) Entgegenhaltungen:
- DE-A1-102012 209 523
- US-A1- 2014 048 785
- US-A1- 2014 107 364

## Beschreibung

Die Erfindung betrifft ein organisches elektronisches Bauteil. Es wird weiterhin ein Verfahren zur Herstellung eines organischen elektronischen Bauteils beschrieben, wobei die organische elektronische Schicht mittels Gasphasenabscheidung erhalten wird.

Die organische Elektronik beschäftigt sich mit Anwendungen organischer Matrixmaterialien zur Umwandlung von Licht in elektrischen Strom und umgekehrt und mit dem Aufbau elektrischer Bauteile mittels organischem Halbleitermaterial. Beispiele für die erstgenannte Kategorie sind etwa Fotodetektoren und organische Solarzellen, schematisch dargestellt in Figur 1, welche Licht in ein elektrisches Signal oder in elektrischen Strom umwandeln und organische Leuchtdioden (OLEDs), welche Licht mittels organischer elektronischer Materialien erzeugen können (siehe Figur 2). In den zweiten Technologiebereich fallen zum Beispiel organische Feldeffekt-Transistoren, schematisch dargestellt in Figur 3, bei denen eine Dotierung den Kontaktwiderstand zwischen Elektrode und Halbleitermaterial verringert oder bipolare Transistoren.

Allen Anwendungen ist gemein, dass sie als wesentliche, funktionale Komponente elektrische Transportschichten beinhalten, welche in Abhängigkeit ihrer Zusammensetzung unterschiedliche Leitungsmechanismen aufweisen. Allgemein unterscheidet man eine intrinsische p-(Loch)- oder eine n-(Elektronen)-Leitfähigkeit der organischen Materialien. Da der Ladungstransport dieser organischen Substanzklassen in der Regel unzureichend ist, werden diese mit zusätzlichen Verbindungen versetzt, welche die Ladunstransporteigenschaften der Schichten verbessern sollen. Üblicherweise erfolgt dies durch Dotieren mit metallischen oder weiteren organischen Verbindungen. Ein Ansatz zur Erzielung signifikanter Verbesserungen der Leitfähigkeiten ist der Zusatz von Metallkomplexen. So wurde beispielsweise von Erfindern der vorliegenden Erfindung bereits in WO 2013/182389 A2, US 2014/0048785 A1 und in WO 2011/033023 A1 die Verwendung von Bismut- und Kupferkomplexen als p-Dotanden für organische elektronische Bauelemente beschrieben.

Die organischen Schichten der beschriebenen Bauelemente, welche ein Matrixmaterial gemeinsam mit dem jeweiligen Metallkomplex als p-Dotand enthalten, wurden dabei beispielsweise über Lösungsmittelprozesse also Nassprozesstechniken erhalten. Außerdem wurden in den genannten Patentschriften organische Schichten auch mittels Gasphasenabscheidung unter Einsatz von Punktquellen erzeugt. Beispiele für Nassprozesstechniken sind insbesondere Drucktechniken wie Tintenstrahl, Tiefdruck und Offsetdruck. Zudem sind auch Spin- und Slotcoating typische Lösungsmittelprozesse.

Die Erzeugung von Schichten organischer elektronischer Bauelemente über Vakuumprozesse erfolgt dagegen mittels Sublimation, also durch thermische Verdampfung. Hier werden die organischen Schichten aus der Gasphase auf einem Substrat oder einer bereits bestehenden Schicht abgeschieden.

Durch letztgenannten Prozess werden derzeit die effizientesten organischen Bauelemente hergestellt, welche inzwischen auch durch eine Pilotproduktion kommerziell verfügbar sind. Die Effizienz der organischen elektronischen Bauelemente wird u.a. auch dadurch erreicht, dass die Bauelemente aus sehr vielen einzelnen Schichten aufgebaut sind. Jede der Schichten besitzt eine spezielle auch auf den Ort im Bauteil bezogene physikalisch elektrische Funktion.

Bei der Herstellung der organischen Schichten durch Abscheiden aus der Gasphase werden das Matrixmaterial und der Dotierstoff beispielsweise durch Koverdampfung bevorzugt aus verschiedenen Quellen auf ein Substrat oder eine bereits bestehende Schicht abgeschieden.

Hierzu werden herkömmlicher Weise Punktquellen eingesetzt. Beispielsweise wurde von Erfindern der vorliegenden Erfindung in WO 2011/033023 A1 und WO 2013/182389 A2 eine Gasphasenabscheidung dotierter organischer Schichten jeweils mittels Punktquellen vorgenommen.

Bei einer Abscheidung aus Punktquellen wird das abzuscheidende Material in einem Tiegel unter Vakuumbedingungen verdampft. Nach dem Verdampfen des Materials treffen die Moleküle aufgrund der hohen mittleren freien Weglänge im Vakuum (10⁻⁵ bis 10⁻⁶ mbar) ohne weitere Stöße auf das Substrat. Dies bedeutet, dass ein Material thermisch nur wenig über die Sublimationstemperatur hinaus stabil sein muss, um unzersetzt auf dem Substrat abgeschieden werden zu können.

Beide Techniken, Nassprozesstechniken wie auch eine Abscheidung aus der Gasphase, die über Punktquellen erfolgt, sind vergleichsweise schonende Techniken. Sie sind daher für eine Vielzahl verschiedener Metallkomplexe einsetzbar.

Obgleich sowohl Nassprozesstechniken wie eine Gasphasenabscheidung mittels Punktquellen eine Herstellung von organischen elektronischen Bauelementen auch unter industriellen Bedingungen erlauben, sind die genannten Techniken insbesondere für die Beschichtung großflächiger Substrate nur bedingt geeignet.

Es wird daher ein weiteres Verfahren für die Herstellung organisch elektronischer Bauteile angegeben, insbesondere ein Verfahren, dass sich auch für die Beschichtung großflächiger Substrate eignet.

Demgemäß wird ein Verfahren zur Herstellung eines organischen elektronischen Bauteils vorgeschlagen, wobei das Bauteil zumindest eine organische elektronische Schicht umfasst. Die organische elektronische Schicht weist dabei eine Matrix auf, wobei die Matrix als Dotand einen Metallkomplex enthält. Beispielsweise kann es sich bei besagtem Dotanden um einen p-Dotand handeln. Besagter Metallkomplex umfasst mindestens ein Metallatom M und mindestens einen an das Metallatom M gebundenen Liganden L, wobei die Liganden L unabhängig voneinander die folgende allgemeine Struktur aufweisen:

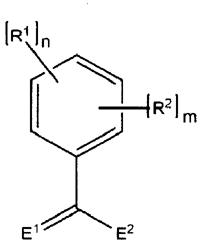

E¹ und E² sind dabei unabhängig voneinander Sauerstoff, Schwefel, Selen, NH oder NR', wobei R' ausgewählt sein kann aus der Gruppe enthaltend Alkyl oder Aryl und mit dem substituierten Benzolring des Liganden L verbunden sein kann.

Die Substituenten R¹ sind unabhängig voneinander ausgewählt aus der Gruppe umfassend verzweigte oder unverzweigte, fluorierte aliphatische Kohlenwasserstoffe mit 1 bis 10 C-Atomen, wobei n = 1 bis 5 ist. Es ist also beispielsweise möglich, dass ein bis fünf Substituenten R¹ vorliegen können, die jeweils unabhängig voneinander aus der Gruppe umfassend verzweigte oder unverzweigte, fluorierte aliphatische Kohlenwasserstoffe mit 1 bis 10 C-Atomen ausgewählt sein können. Es können dabei also beispielsweise mehrere gleiche aber auch mehrere verschiedene Substituenten R¹ vorliegen.

Die Substituenten R² sind unabhängig voneinander ausgewählt aus der Gruppe umfassend -CN, verzweigte oder unverzweigte aliphatische Kohlenwasserstoffe mit 1 bis 10 C-Atomen, Aryl und Heteroaryl, wobei m = 0 bis maximal 5-n ist. Es ist also beispielsweise möglich, dass ein oder mehrere Substituenten R² vorliegen, die unabhängig voneinander ausgewählt sind aus der Gruppe umfassend -CN, verzweigte oder unverzweigte aliphatische Kohlenwasserstoffe mit 1 bis 10 C-Atomen, Aryl und Heteroaryl. Es ist aber beispielsweise auch möglich, dass der Ligand L keinen Substituenten R² aufweist.

Während der Ligand L also in jedem Falle zumindest einen Substituenten R¹ aufweist, muss er nicht unbedingt einen Substituenten R² aufweisen.

Alle nicht substituierten Positionen des Benzolringes des Liganden L sind mit Wasserstoff oder Deuterium besetzt.

Das Metall M kann beispielsweise ein Hauptgruppenmetall oder ein Übergangsmetall sein.

Die Abscheidung des Dotanden der zumindest einen organischen elektronischen Schicht erfolgt mittels einer Quelle für Gasphasenabscheidungen, wobei die Quelle so ausgestaltet ist, dass der Dotand Stöße mit zumindest einer Wand der Quelle erfährt.

Im Gegensatz zu einer Quelle wie sie wie hier beschrieben verwendet wird, sind bei einer Punktquelle der Bereich der Quelle, in welchem der Dotand verdampft wird, die Austrittsöffnung der Quelle, sowie das Substrat, auf dem der Dotand abgeschieden wird, alle in einer geraden Linie angeordnet, so dass der Dotand direkten Zugang zum Substrat hat und Stöße mit Wänden der Quelle vermieden werden können.

Bei einer Quelle wie sie bei dem beschriebenen Verfahren zum Einsatz kommt, sind der Bereich der Quelle, in welchem der Dotand verdampft wird, sowie die Austrittsöffnung der Quelle und das Substrat nicht in einer geraden Linie angeordnet. Die Quelle zeichnet sich vielmehr dadurch aus, dass der Dotand, nachdem er in einem Bereich der Quelle verdampft wurde, noch innerhalb der Quelle eine Umlenkung erfährt, bevor er die Quelle verlässt und auf das Substrat trifft.

Beispielsweise kann der Dotand dabei Stöße mit mehreren Wänden der Quelle erfahren. Beispielsweise kann der Gasstrom mit dem Dotand über Bereiche der Quelle etwa in Form von Rohren geleitet werden, wobei der Dotand zahlreiche Stöße mit Wänden der Quelle erfährt. Beispielsweise können die Wände der Quelle beheizt sein, so dass sie eine Temperatur aufweisen, die mindestens so hoch ist wie die Sublimationstemperatur des Dotanden.

Derartige Quellen haben gegenüber herkömmlichen Quellen für die Gasphasenabscheidung den Vorteil, dass sie eine bessere Führung des Gasstromes erlauben. Dies kann insbesondere für die Gasphasenabscheidung bei zu beschichtenden großflächigen Substraten von Vorteil sein.

Bei dem hier beschriebenen Verfahren kann der Dotand beispielsweise mehrere tausend Stöße mit den Wänden der Quelle erfahren, bevor er die Quelle verlässt, ohne sich dabei jedoch zu zersetzen.

Bei vielen Quellen erleidet der abzuscheidende Dotand mehrere tausend Stöße mit den Wänden der Quelle, bis er über eine Austrittsöffnung beispielsweise in Form eines Spalts oder mehrerer Löcher ins freie Vakuum tritt. Um an den Wänden der Quelle eine Materialabscheidung zu verhindern werden selbige häufig auf Temperaturen von 20-80 Kelvin oder sogar deutlich höher über dem eigentlichen Sublimationspunkt des abzuscheidenden Dotanden erwärmt. Diesen Temperaturen muss der Dotand bei der Abscheidung aus der Gasphase standhalten können, ohne sich zu zersetzen.

Die Erfinder der vorliegenden Erfindung haben festgestellt, dass herkömmliche Metallkomplexe, die als Dotanden in organischen Schichten zum Einsatz kommen zwar stabil genug für eine Verarbeitung mittels Nassprozesstechniken oder einer Gasphasenabscheidung über Punktquellen sind, jedoch nicht ausreichend stabil um mittels Quellen abgeschieden zu werden, wobei der Dotand Stöße mit zumindest einer Wand der Quelle erfährt.

Beispielsweise haben die Erfinder, die auch das vorliegende Verfahren beschreiben, beobachtet, dass die p-Dotanden von WO 2013/182389 A2 und WO 2011/033023 A1 im Allgemeinen zwar mittels Lösungsmittel-prozessen und durch Gasphasenabscheidung aus Punktquellen gemeinsam mit geeigneten Matrixmaterialien in Form organischer Schichten abgeschieden werden können, jedoch viele der darin genannten Verbindungen nicht hinreichend temperaturstabil für eine Abscheidung mittels Quellen sind, in welchen der Dotand Stöße mit Wänden der Quelle erfährt.

Beispielsweise haben die Erfinder der vorliegenden Erfindung erkannt, dass Metallkomplexe z.B. des Kupfers oder Bismuts nicht hinreichend stabil für eine Abscheidung sind, wenn sie keine Substituenten der Form R¹ aufweisen. Beispielsweise sind Benzoesäurederivate mit fluoriertem Benzolring aber ohne Substituenten der Form von R¹ nicht hinreichend stabil um im beschriebenen Verfahren Verwendung zu finden.

Die Erfinder der vorliegenden Erfindung haben erkannt, dass Metallkomplexe wie sie beim beschriebenen Verfahren zum Einsatz kommen überraschenderweise eine hinreichende thermische Stabilität aufweisen, um mittels einer Quelle verdampft und abgeschieden zu werden, wobei die Quelle so ausgestaltet ist, dass der Dotand Stöße mit zumindest einer Wand der Quelle erfährt.

So haben die Erfinder insbesondere festgestellt, dass die Einführung von zumindest einem Substituenten R¹ bei dem es sich um einen verzweigten oder unverzweigten, fluorierten aliphatischen Kohlenwasserstoff mit 1 bis 10 C-Atomen handelt eine deutliche Verbesserung der thermischen Stabilität des gesamten Komplexes erreicht werden kann, wodurch eine Abscheidung über Quellen gemäß Anspruch 1 erst ermöglicht wird.

Gleichzeitig zeichnen sich die Metallkomplexe wie sie beim beschriebenen Verfahren zum Einsatz kommen neben einer hohen Temperaturstabilität zugleich durch ausreichend gute Dotierstärken aus.

Schichten von organischen elektronischen Bauelementen, die mit dem beschriebenen Verfahren erzeugt werden, zeichnen sich zudem durch eine hohe optische Transparenz im sichtbaren Bereich aus.

Die in dem beschriebenen Verfahren verwendeten Metallkomplexe sind zu vergleichbaren Preisen erhältlich wie herkömmliche Metallkomplexe und sind zudem ohne großen technischen Aufwand herstellbar.

Der Einsatz von Quellen in welchen der Dotand Stöße mit zumindest einer Wand der Quelle erfährt erlaubt insgesamt eine weniger aufwendige, zeitsparende und kostengünstigere Anfertigung organischer elektronischer Bauteile. Das beschriebene Verfahren eignet sich daher besonders gut für eine Anwendung im industriellen Maßstab. Insbesondere für die Beschichtung großflächiger Substrate ist das beschriebene Verfahren deutlich besser geeignet als herkömmliche Verfahren, bei welchen beispielsweise Punkt-Quellen zum Einsatz kommen. Bei dem beschriebenen Verfahren handelt es sich also um eine großflächige Beschichtungsmethode.

Im Folgenden sei kurz auf einige Begriffsdefinitionen verwiesen:
Im Sinne der vorliegenden Erfindung bedeutet und/oder umfasst die Bezeichnung "organisches elektronisches Bauteil" insbesondere organische Transistoren, organische lichtemittierende Dioden, lichtemittierende elektrochemische Zellen, organische Solarzellen, Photodioden sowie organische Photovoltaik allgemein.

Im Sinne der vorliegenden Erfindung umfasst oder bedeutet die Bezeichnung "p-Dotand" insbesondere Materialien, die eine Lewis-Acidität aufweisen und /oder in der Lage sind, Komplexe mit dem Matrixmaterial auszubilden, in denen diese Materialien (wenn auch nur formal) Lewis-azide wirken.

Im Folgenden wird auf eine Reihe von bevorzugten Beispielen des beschriebenen Verfahrens eingegangen:
Gemäß einem besonders bevorzugten Beispiel kommt beim beschriebenen Verfahren eine Linearquelle bei der Gasphasenabscheidung zum Einsatz.

Bei Linearquellen erfolgt die Abscheidung aus der Gasphase über einen Spalt als Austrittsöffnung etwa in Form einer Lochreihe. Hier erleidet das Molekül oft mehrere tausend Stöße mit den Wänden der Quelle, bis es schließlich durch den Spalt oder mehrere Löcher ins freie Vakuum tritt. Um an den Wänden der Linearquelle eine Materialabscheidung zu verhindern werden diese oft auf Temperaturen von 20-80 Kelvin über dem eigentlichen Sublimationspunkt geheizt. Auch ein Heizen auf noch höhere Temperaturen ist möglich.

Herkömmliche Dotanden zersetzten sich meist bei den Stößen mit den Wänden der Quelle. Beispielsweise haben die Erfinder erkannt, dass viele der Metallkomplexe von WO 2013/182389 A2 und WO 2011/033023 A1, zwar stabil genug für eine Abscheidung mittels Punktquellen sind, sich jedoch wie experimentell gezeigt nicht mittels Linearquellen abscheiden lassen, in welchen die Komplexe Stößen mit den Wänden der Quelle ausgesetzt sind.

Auch die in dem Artikel von Schmid et al. "Fluorinated Copper(I) Carboxylates as Advanced Tunable p-Dopants for Organic Light-Emitting Diodes" (Advanced Materials 2014, Vol. 26, 6, 878-885) beschriebenen Verbindungen sind p-Dotierstoffe. Zwar konnten die dort beschriebenen Verbindungen von Erfindern der vorliegenden Erfindung unzersetzt in einer Punktquelle verdampft und charakterisiert werden, ein Transfer auf eine Linearquelle war jedoch nicht möglich.

Gemäß einem bevorzugten Beispiel wird bei dem beschriebenen Verfahren ein Metallkomplex der beschriebenen Form verwendet, der Lewis-sauer ist, d.h. er agiert als Elektronenpaarakzeptor. Dies hat sich für eine Interaktion mit den Matrixmaterialien als besonders bevorzugt herausgestellt.

Ein Beispiel betrifft das beschriebene Verfahren, wobei der Metallkomplex mehrere gleiche Liganden L aufweist. Solche Komplexe sind meist einfacher herzustellen als Metallkomplexe mit verschiedenartigen Liganden L.

Ein Beispiel betrifft das beschriebene Verfahren, wobei der Metallkomplex zumindest zwei verschiedenartige Liganden L aufweist. Auch solche Komplexe können in Quellen, die so ausgestaltet sind, dass es zu Stößen des Dotand mit zumindest einer Wand der Quelle kommt abgeschieden werden.

Ein Beispiel betrifft das beschriebene Verfahren, wobei der Metallkomplex neben dem Liganden L noch andere Liganden einer anderen von L verschiedenen Formel aufweist. Auch solche Komplexe sind durch eine hohe thermische Stabilität gekennzeichnet.

Gemäß einem anderen Beispiel wird bei dem beschriebenen Verfahren ein Metallkomplex verwendet, der mindestens eine offene oder teilweise zugängliche Koordinationsstelle aufweist. Dies hat sich ebenfalls für eine Interaktion mit den Matrixmaterialien als besonders bevorzugt herausgestellt.

Ein bevorzugtes Beispiel betrifft das beschriebene Verfahren, wobei das Metall M des Metallkomplexes ausgewählt ist aus der Gruppe der Hauptgruppenmetalle und Übergangsmetalle. Insbesondere kann es sich bei dem Metall M um die Hauptgruppenmetalle der Gruppen 13 bis 15 des Periodensystems und die Metalle Cu, Cr, Mo, Rh und Ru handeln.

Diese Komplexe haben sich als p-Dotanden in organischen Schichten von organischen elektronischen Bauelementen bewährt. Aufgrund ihrer Lewis-Azidität werden mit den Komplexen der besagten Metalle gute p-Dotierstoffwirkungen erzielt. Außerdem sind die beschriebenen Metallkomplexe leicht herstellbar und erfordern keine aufwendigen Herstellungsverfahren. Die Leitfähigkeit organischer Schichten lässt sich zudem einfach über die Konzentration der Dotierstoffe besagter Metalle den jeweiligen Erfordernissen anpassen.

Eine anderes Beispiel betrifft das beschriebene Verfahren, wobei das Metall M des Metallkomplexes Bismut oder Kupfer ist. Beispielsweise kann es sich um einen Metallkomplex mit Bismut in den Oxidationsstufen III oder V handeln. Besonders bevorzugt sind dabei Bismut komplexe in der Oxidationsstufe III. Beispielsweise kann der Metallkomplex ein Kupfer(I) oder ein Kupfer(II)-Komplex sein. Besonders bevorzugt sind dabei Kupferkomplexe in der Oxidationsstufe I.

Metallkomplexe des Kupfers und Bismuts haben sich als besonders effektive p-Dotierstoffe bewährt, die einfach herzustellen sind. Beispielsweise zeichnen sich organische elektronische Bauelemente umfassend Lochtransport-Schichten mit den besagten Dotierstoffen durch besonders gute Leitfähigkeiten aus. Zudem sind entsprechende Komplexe besonders thermisch stabil.

In einer besonders bevorzugten Ausführungsform handelt es sich bei dem Substituenten R¹ um einen zumindest zweifach Fluorierten Substituenten, d.h. R¹ weist zumindestens zwei Fluoratome auf. Noch weiter bevorzugt ist es, wenn der Substituent R¹ ein perfluorierter Substituent ist. Je höher der Fluorierungsgrad, umso stärker der stabilisierende Effekt des Substituenten auf den Metallkomplex.

Ein besonders bevorzugtes Beispiel betrifft das beschriebene Verfahren, wobei zumindest einer der Substituenten R¹ des Liganden L im Metallkomplex, eine -CF₃ Gruppe ist.

Die Erfinder der vorliegenden Erfindung haben erkannt, dass das beschriebene Verfahren mit dieser Art von Metallkomplexen besonders zu bevorzugen ist, da derartige Metallkomplexe eine besonders hohe thermische Stabilität aufweisen und sich daher auch beim Einsatz in Quellen bei welchen es zu Stößen des Metallkomplexes mit zumindest einer Wand der Quelle kommt nicht zersetzten.

Die Erfinder haben beobachtet, dass die elektronischen und sterischen Eigenschaften der -CF₃ Gruppe als Substituent R¹ des Liganden L zu besonders stabilen Metallkomplexen führen. Insbesondere werden sehr hohe thermische Stabilitäten ermöglicht, so dass sich Metallkomplexe, mit -CF₃ Gruppe als Substituent R¹ des Liganden L, erst bei Temperaturen weit über dem Sublimationspunkt zersetzen.

Auch begünstigt die -CF₃ Gruppe eine höhere Lewis-Azidität des Metallkomplexes und führt zu besonders guten Dotierstoffstärken, was die Leitfähigkeit in der organischen Schicht des hergestellten organischen elektronischen Bauelements begünstigt.

Zudem sind Liganden mit -CF₃ Substituenten im Vergleich zu anderen fluorierten aliphatischen Kohlenwasserstoffen in Ausgangsmaterialien für die Herstellung des Liganden L weiter verbreitet, so dass Liganden bei welchen der Substituent R¹ eine -CF₃ Gruppe ist, häufig leichter zugänglich und preiswerter sind als Liganden mit anderen Substituenten R¹.

Ein anderes Beispiel betrifft das beschriebene Verfahren, wobei der der Ligand L genau zwei Substituenten R¹ aufweist, die jeweils eine -CF₃ Gruppe bilden.

Die Erfinder der vorliegenden Erfindung haben erkannt, dass überraschender Weise durch den Einsatz von zwei -CF₃ Gruppen im Gegensatz zur Verwendung von beispielsweise nur einer -CF₃ Gruppe eine weitere Verbesserung der Temperaturbeständigkeit des Metallkomplexes erreicht werden kann. Dies führt zu besonders guten Ergebnissen bei der Aufbringung der organischen Schicht umfassend den besagten Metallkomplex als Dotierstoff mittels Quellen, in welchen der Dotierstoff, also der Dotand Stöße mit zumindest einer der Wände der Quellen erfährt.

Ein anderes Beispiel betrifft das beschriebene Verfahren, wobei der Ligand L genau zwei Substituenten R¹ aufweist, die jeweils eine -CF₃ Gruppe bilden und in 3,5-Position am Benzolring des Liganden L angeordnet sind.

Die Erfinder der vorliegenden Erfindung haben erkannt, dass Komplexe mit Liganden L der besagten Konnektivität eine besonders hohe thermische Stabilität erlauben. Die Erfinder haben festgestellt, dass durch den sterischen Anspruch der in 3,5-Position am Benzolring befindlichen Gruppen eine besonders gute Stabilisierung des Metallkomplexes möglich ist.

Ein weiteres Beispiel betrifft das beschriebene Verfahren, wobei die Substituenten R² unabhängig voneinander ausgewählt sind aus der Gruppe umfassend -CN, Methyl-, Ethyl-, n-Propyl-, iso-Propyl-, n-Butyl-, iso-Butyl-, tert-Butyl- und substituiertes oder unsubstituiertes Phenyl-.

Die Erfinder der vorliegenden Erfindung haben erkannt, dass eine Verdampfung derartiger Komplexe mittels Quellen bei denen der Dotand Stöße mit zumindest einer der Wände der Quelle erfährt, ohne Zersetzung des Komplexes möglich ist. Es können also auch Komplexe mit Liganden L in Linearquellen verwendet werden, bei welchen nicht alle Substituenten fluorierte Kohlenwasserstoffe sind.

Ein weiteres Beispiel betrifft das beschriebene Verfahren, bei welchem der Ligand L des Metallkomplexes keinen Substituenten R² aufweist, d.h. m=0.

Da unfluorierte Kohlenwasserstoff-Substituenten eine höhere Reaktivität besitzen als fluorierte Kohlenwasserstoff-Substituenten, führt ein Verzicht auf einen Substituenten R² zu einer weiteren Verbesserung der Stabilität und damit besseren Abscheidbarkeit aus der Gasphase mittels Quellen in denen Stöße des Metallkomplexes mit zumindest einer der Wände der Quelle erfolgen.

In einer anderen bevorzugten Weiterbildung der Erfindung weist der Metallkomplex genau zwei Substituenten R¹ auf oder noch mehr Substituenten R¹ auf. Zwei oder mehr Substituenten R¹ führen zu einer besonders guten Abschirmung des Komplexes nach außen und gestatten daher eine besonders gute Stabilisierung des Metallkomplexes.

Ein anderes Beispiel betrifft das beschriebene Verfahren, wobei sowohl E¹ als auch E² des Liganden L des Metallkomplexes Sauerstoff sind. In diesem Fall handelt es sich bei dem Ligand L um ein mit fluorierten Kohlenwasserstoffen substituiertes Derivat des Benzoat.

Die Erfinder der vorliegenden Erfindung haben festgestellt, dass derartige Metallkomplexe besonders gut für die Herstellung von organischen elektronischen Bauelementen mittels Abscheidung über Quellen, bei welchen der Dotand Stöße mit zumindest einer der Wände der Quelle erfährt, geeignet sind, da sie leicht herstellbar sind, Metallkomplexe guter Dotierstoffstärken ergeben und besonders hohen Anforderungen an die thermische Stabilität genügen. Insbesondere sind Derivate der Benzoesäure oftmals gut kommerziell erhältlich oder ohne hohen technischen Aufwand herzustellen.

Ein besonders bevorzugtes Beispiel betrifft das beschriebene Verfahren, wobei der Ligand L des Metallkomplexes unabhängig voneinander ausgewählt ist aus der Gruppe umfassend

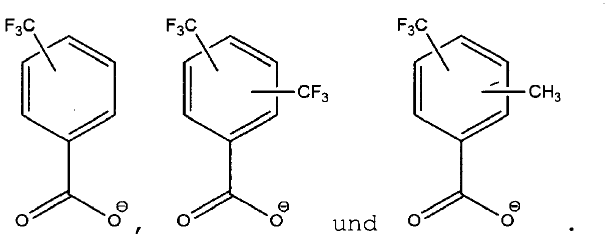

Falls mehrere Liganden L vorliegen können dabei alle Liganden L des Metallkomplexes unabhängig voneinander ausgewählt sein. Beispielsweise können mehrere verschiedene der genannten Liganden L im Metallkomplex vorliegen oder es können auch alle Liganden L gleich sein.

Die Erfinder der vorliegenden Erfindung haben festgestellt, dass Metallkomplexe mit Liganden L aus dieser Gruppe eine besonders gute Temperaturbeständigkeit aufweisen und zudem besonders gute Dotierstoffeigenschaften besitzen. Es hat sich überraschender Weise herausgestellt, dass diese Metallkomplexe in besonderer Weise für die Abscheidung mittels Quellen geeignet sind, bei welchen die Metallkomplexe Stöße mit zumindest einer Wand der Quelle erfahren und sich dennoch nicht in der Quelle zersetzen.

Ein weiteres bevorzugtes Beispiel betrifft das beschriebene Verfahren, wobei der Ligand L des Metallkomplexes unabhängig voneinander ausgewählt ist aus der Gruppe umfassend

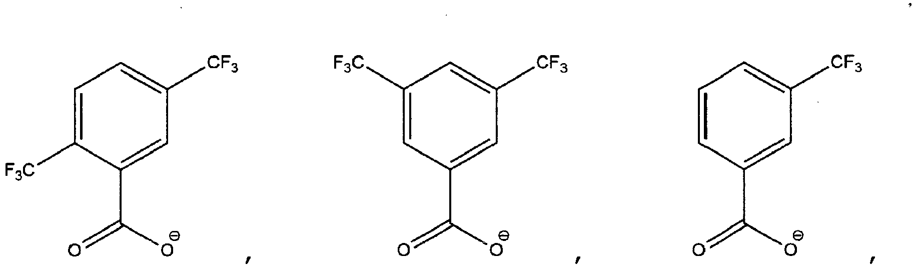

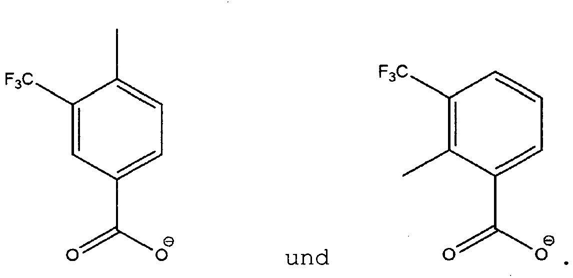

Die genannten Beispiele für den Liganden L sind kommerziell zu vertretbaren Preisen verfügbar und müssen daher nicht in Eigenproduktion hergestellt werden. Sie eignen sich daher auch besonders für Anwendungen im industriellen Maßstab.

Ein noch weiter bevorzugtes Beispiel betrifft das beschriebene Verfahren, wobei es sich bei dem Ligand L des Metallkomplexes um 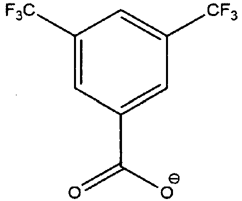 handelt.

Durch die Verwendung eines Derivates der Benzoesäure, mit zwei -CF₃ Substituenten in 3,5-Position werden Metallkomplexe erhalten, die noch weit über der Sublimationstemperatur thermisch stabil sind. Diese Komplexe sind besonders gut für ein Verfahren mit einer Abscheidung über Quellen, bei welchen es zu Stößen mit zumindest einer der Wände der Quellen kommt, beispielsweise Linearquellen, geeignet. Zudem ergeben sie organische elektronische Bauelemente mit ausgezeichneten elektrischen Eigenschaften.

In einem anderen Beispiel des beschriebenen Verfahrens handelt es sich bei dem verwendeten Metallkomplex um einen mononuklearen Metallkomplex, also um einen Metallkomplex mit nur einem Zentralatom.

In einem davon verschiedenen Beispiel des beschriebenen Verfahrens kommen mehrkernige Metallkomplexe zum Einsatz. Auf diese Weise kann häufig durch die Verfügbarkeit mehrerer Lewis-saurer Zentren die Leitfähigkeit der abzuscheidenden organischen Schichten noch besser eingestellt werden.

Ein weiteres Beispiel betrifft das beschriebene Verfahren bei welchem nur homoleptische Metallkomplexe zum Einsatz kommen. Derartige Komplexe sind oft weniger aufwendig herzustellen, da sie nur eine Art von Liganden der Formel des Liganden L umfassen. Es werden so auch keine anderen Liganden eingeführt, die potentiell die Stabilität des gesamten Komplexes mindern können.

Ein anderes Beispiel betrifft das beschriebene Verfahren, wobei der Ligand L unabhängig voneinander durch eine der folgenden Formen der Koordination an das Metallatom M des Metallkomplexes gebunden ist:

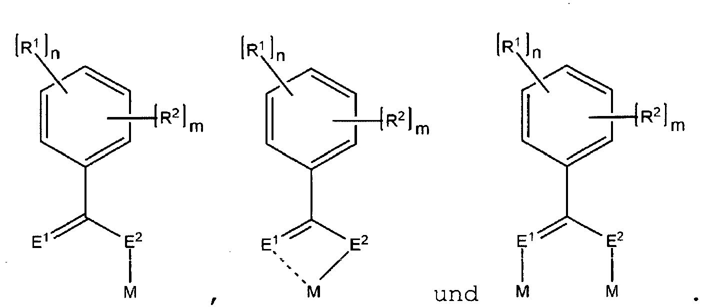

Experimentelle Befunde der Erfinder zeigen, dass die Bindung zum Metallatom M vielfältig sein kann. Die Liganden können einzähnig, zweizähnig oder verbrückend gebunden sein, um die Erhöhung der Temperaturstabilität zu bewirken.

Ein besonders bevorzugtes Beispiel betrifft das beschriebene Verfahren, wobei der Metallkomplex ein Bismutkomplex ist und wobei der Ligand L unabhängig voneinander die folgende allgemeine Struktur aufweisen kann: 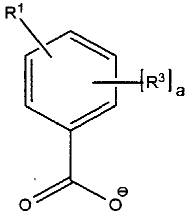
wobei der Substituent R¹ ausgewählt ist aus der Gruppe umfassend verzweigte oder unverzweigte, fluorierte aliphatische Kohlenwasserstoffe mit 1 bis 10 C-Atomen, und
wobei der Substituent R³ ausgewählt ist aus der Gruppe umfassend verzweigte oder unverzweigte fluorierte oder nicht fluorierte aliphatische Kohlenwasserstoffe mit 1 bis 10 C-Atomen, Aryl und Heteroaryl, wobei a gleich 0 oder 1 sein kann.

Die Erfinder der vorliegenden Erfindung haben erkannt, dass Bismutkomplexe mit den besagten Liganden eine besonders hohe Temperaturbeständigkeit aufweisen. Daher können besagte Komplexe besonders gut mittels Quellen abgeschieden werden in welchen es zu Stößen mit zumindest einer der Wände der Quelle kommt, weshalb das Verfahren eine Produktion von organischen elektronischen Bauteilen mit besonders geringem technischem Aufwand erlaubt und somit besonders kostengünstig ist.

Ein weiteres Beispiel betrifft das beschriebene Verfahren, wobei der Metallkomplex eine Zersetzungstemperatur aufweist, die höher als 10 Kelvin, weiterhin höher als 20 Kelvin und insbesondere über 40 Kelvin über der Sublimationstemperatur des Metallkomplexes liegt. Am meisten bevorzugt liegt die Zersetzungstemperatur über 70 Kelvin über der Sublimationstemperatur des Metallkomplexes.

Je weiter die Zersetzungstemperatur über der Sublimationstemperatur des Metallkomplexes liegt, umso stabiler sind die Metallkomplexe gegenüber Stößen mit den Wänden der Quelle.

Ein weiteres Beispiel betrifft das beschriebene Verfahren, wobei das Matrixmaterial der organischen elektronischen Schicht, welches beispielsweise mittels Koverdampfung gemeinsam mit dem Metallkomplex abgeschieden wird, ausgewählt ist aus der Gruppe umfassend oder bestehend aus einem oder mehreren der folgenden Materialien, die beispielsweise in Lochtransport-Schichten Verwendung finden können:
NPB (N,N'-Bis(naphthalen-1-yl)-N,N'-bis(phenyl)-benzidin),
β-NPB N,N'-Bis(naphthalen-2-yl)-N,N'-bis(phenyl)-benzidin)
TPD (N,N'-Bis(3-methylphenyl)-N,N'-bis(phenyl)-benzidin)
Spiro TPD (N,N'-Bis(3-methylphenyl)-N,N'-bis(phenyl)-benzidin)
Spiro-NPB (N,N'-Bis(naphthalen-1-yl)-N,N'-bis(phenyl)-spiro)
DMFL-TPD N,N'-Bis(3-methylphenyl)-N,N'-bis(phenyl)-9,9-dimethyl-fluoren)
N,N'-Bis(naphthalen-1-yl)-N,N'-bis(phenyl)-2,2-dimethylbenzidin
N,N'-Bis(3-methylphenyl)-N,N'-bis(phenyl)-9,9-spirofluoren
N,N'-Bis(naphthalen-1-yl)-N,N'-bis(phenyl)-9,9-spirofluoren
DMFL-NPB (N,N'-Bis(naphthalen-1-yl)-N,N'-bis(phenyl)-9,9-dimethyl-fluoren)
DPFL-TPD (N,N'-Bis(3-methylphenyl)-N,N'-bis(phenyl)-9,9-diphenyl-fluoren)
DPFL-NPB (N,N'-Bis(naphthalen-1-yl)-N,N'-bis(phenyl)-9,9-diphenyl-fluoren)
Spiro-TAD (2,2',7,7'-Tetrakis(N,N-diphenylamino)-9,9'-spirobifluoren)
9,9-Bis[4-(N,N-bis-biphenyl-4-yl-amino)phenyl]-9H-fluoren
9,9-Bis[4-(N,N-bis-naphthalen-2-yl-amino)phenyl]-9H-fluoren
9,9-Bis[4-(N,N'-bis-naphthalen-2-yl-N,N'-bis-phenyl-amino)-phenyl]-9H-fluoren
xN,N'-bis(phenanthren-9-yl)-N,N'-bis(phenyl)-benzidin
2,7-Bis[N,N-bis(9,9-spiro-bifluorene-2-yl)-amino]-9,9-spiro-bifluoren
2,2'-Bis[N,N-bis(biphenyl-4-yl)amino]9,9-spiro-bifluoren
2,2'-Bis(N,N-di-phenyl-amino)9,9-spiro-bifluoren
Di-[4-(N,N-ditolyl-amino)-phenyl]cyclohexan
2,2',7,7'-tetra(N,N-di-tolyl)amino-spiro-bifluoren
N,N,N',N'-tetra-naphthalen-2-yl-benzidin
2,2',7,7'-tetrakis[N-naphthalenyl(phenyl)-amino]-9,9-spirobifluoren
Spiro-TTB (2,2',7,7'-Tetrakis-(N,N'-di-p-methylphenylamino)-9,9'-spirobifluoren)
Titanoxid-phthalocyanin
Kupfer-phthalocyanin
2,3,5,6-Tetrafluoro-7,7,8,8-tetracyano-quinodimethan
4,4',4"-Tris(N-3-methylphenyl-N-phenyl-amino)triphenylamin
4,4',4"-Tris(N-(2-naphthyl)-N-phenyl-amino)triphenylamin
4,4',4"-Tris(N-(1-naphthyl)-N-phenyl-amino)triphenylamin
4,4',4"-Tris(N,N-diphenyl-amino)triphenylamin
Pyrazino[2,3-f][1,10]phenanthroline-2,3-dicarbonitril
N,N,N',N'-Tetrakis(4-methoxyphenyl)benzidin

Diese Materialien haben sich als Matrixmaterialien in organischen elektronischen Bauteilen bewährt.

Ein weiteres Beispiel betrifft das beschriebene Verfahren, wobei die zumindest eine organische elektronische Schicht des mit dem Verfahren herzustellenden organischen elektronischen Bauelements eine elektronenblockierende Schicht ist. Hierbei erfolgt die Koverdampfung mittels Quellen wobei es zu Stößen mit zumindest einer Wand der Quelle kommt, beispielsweise mit Linearquellen, mit zumindest teilweise elektronenleitenden Matrixmaterialien.

Typische elektronenleitende Materialien sind hierbei:
2,2',2"-(1,3,5-Benzinetriyl)tris(1-phenyl-1-H-benzimidazol)
2-(4-Biphenylyl)-5-(4-tert-butylphenyl)-1,3,4-oxadiazol
2,9-Dimethyl-4,7-diphenyl-1,10-phenanthrolin
8-Hydroxyquinolinolato-lithium
4-(Naphthalen-1-yl)-3,5-diphenyl-4H-1,2,4-triazol
1,3-Bis[2-(2,2'-bipyridin-6-yl)-1,3,4-oxadiazo-5-yl]benzene
4,7-Diphenyl-1,10-phenanthrolin
3-(4-Biphenylyl)-4-phenyl-5-tert-butylphenyl-1,2,4-triazol Bis(2-methyl-8-quinolinolat)-4-(phenylphenolato)aluminium
6,6'-Bis[5-(biphenyl-4-yl)-1,3,4-oxadiazo-2-yl]-2,2'bipyridyl 2-phenyl-9,10-di(naphthalen-2-yl)-anthracen
2,7-Bis[2-(2,2'-bipyridine-6-yl)-1,3,4-oxadiazo-5-yl]-9,9-dimethylfluoren
1,3-Bis[2-(4-tert-butylphenyl)-1,3,4-oxadiazo-5-yl]benzene
2-(naphthalen-2-yl)-4,7-diphenyl-1,10-phenanthrolin
2,9-Bis(naphthalen-2-yl)-4,7-diphenyl-1,10-phenanthrolin Tris(2,4,6-trimethyl-3-(pyridin-3-yl)phenyl)boran
1-methyl-2-(4-(naphthalen-2-yl)phenyl)-1H-imidazo[4,5-f][1,10]phenanthrolin.

Eine Blockierung und Begrenzung des Elektronenflusses ist beispielsweise für hoch effiziente organische lichtemittierende Dioden (OLEDs) von hoher Bedeutung und das beschriebene Verfahren daher von hohem Nutzen im Rahmen einer industriellen Fertigung.

Die Erfindung betrifft ein organisches elektronisches Bauteil umfassend zumindest eine organische elektronische Schicht. Die organische elektronische Schicht umfasst eine Matrix, wobei die Matrix als Dotand, beispielsweise als p-Dotand, einen Bismutkomplex enthält. Der Bismutkomplex umfasst mindestens einen an das Bismutatom gebundenen Liganden L, wobei die Liganden L unabhängig voneinander die folgende allgemeine Struktur aufweisen können: 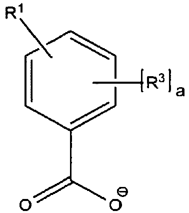 wobei der eine Substituent R¹ ausgewählt ist aus der Gruppe umfassend verzweigte oder unverzweigte, fluorierte aliphatische Kohlenwasserstoffe mit 1 bis 10 C-Atomen, und
wobei der eine Substituent R³ ausgewählt ist aus der Gruppe umfassend verzweigte oder unverzweigte fluorierte oder nicht fluorierte aliphatische Kohlenwasserstoffe mit 1 bis 10 C-Atomen, Aryl und Heteroaryl, wobei a gleich 0 oder 1 sein kann.

Die Erfinder der vorliegenden Erfindung haben festgestellt, dass organische elektronische Bauelemente dieser Form mit deutlich geringerem technischem Aufwand gefertigt werden können als herkömmliche organische elektronische Bauelemente.

Dies ist möglich, weil erfindungsgemäße Bauelemente Bismutkomplexe der gerade beschriebenen Form aufweisen, die sich durch eine besonders hohe Temperaturbeständigkeit auszeichnen. Es ist daher möglich auch Fertigungsverfahren einzusetzen, die eine höhere Temperaturbeständigkeit von den Metallkomplexen erfordern, also ein Fertigungsverfahren bei welchem es in der Quelle für die Gasabscheidung zu Stößen der Komplexe mit zumindest einer Wand der Quelle oder zu Stößen der Komplexe miteinander kommt Beispielsweise ist es möglich eine Fertigung mittels Linearquellen vorzunehmen, die eine besonders hohe thermische Beständigkeit erfordern.

Eine weitere Ausführungsform des erfindungsgemäßen organischen elektronischen Bauteils zeichnet sich dadurch aus, dass der Ligand L unabhängig ausgewählt ist aus der Gruppe umfassend

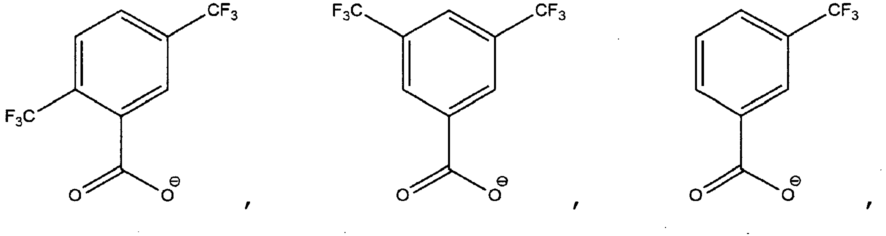

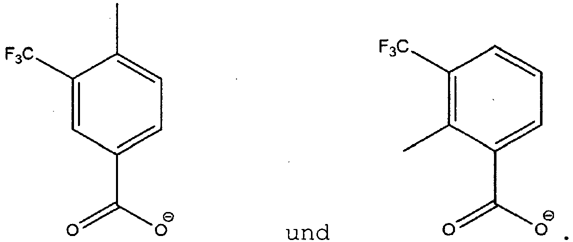

Diese Bauteile sind neben einer besonders einfachen Fertigung aufgrund der Temperaturbeständigkeit der Bismutkomplexe zudem besonders kostengünstig, da die genannten Liganden kommerziell zu vertretbaren Preisen erhältlich sind und somit keine Eigenfertigung erforderlich ist.

Eine besonders bevorzugte Ausführungsform des erfindungsgemäßen organischen elektronischen Bauteils betrifft ein Bauteil, wobei der Ligand L des Bismutkomplexes in den Positionen 3 und 5 des Benzolringes substituiert ist. Solche Komplexe sind besonders stabil.

Eine besonders bevorzugte Ausführungsform des erfindungsgemäßen organischen elektronischen Bauteils betrifft ein Bauteil, wobei der Ligand L des Bismutkomplexes 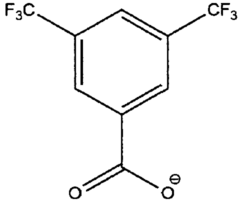 ist.

Eine besonders bevorzugte Ausführungsform des erfindungsgemäßen organischen elektronischen Bauteils betrifft ein Bauteil, wobei der Bismutkomplex der Komplex 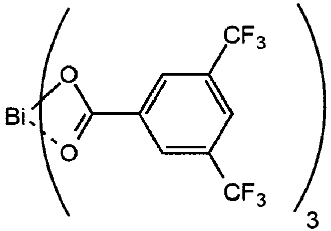 ist.

Die Erfinder der vorliegenden Erfindung haben beobachtet, dass dieser Komplex sowohl gute Dotierstoffeigenschaften zeigt, als auch eine besonders hohe Temperaturstabilität. Der Komplex ist noch bei Temperaturen 70°C über seiner Sublimationstemperatur stabil und daher ausgezeichnet geeignet für Fertigungsverfahren die eine hohe Temperaturbeständigkeit der eingesetzten Dotierstoffe verlangen. Der Komplex ist ausgezeichnet für eine Gasphasenabscheidung auch in Quellen geeignet, die eine besondere Stabilität des Komplexes erfordern, insbesondere Quellen, wobei es zu Stößen mit zumindest einer Wand der Quelle kommt. Beispielsweise kann der Komplex mit geringem Aufwand im Rahmen einer Abscheidung mittels Linearquellen zusammen mit dem Matrixmaterial aufgebracht werden.

Aus diesen Gründen sind organische elektronische Bauelemente umfassend den genannten Komplex auch im großtechnischen Maßstab besonders einfach und kostengünstig herzustellen und besitzen zugleich organische elektronische Schichten mit sehr guten elektrischen Eigenschaften z.B. im Hinblick auf die Leitfähigkeit.

Die vorgenannten sowie die beanspruchten und in den Ausführungsbeispielen beschriebenen erfindungsgemäß zu verwendenden Bauteile unterliegen in ihrer Größe, Formgestaltung, Materialauswahl und technischen Konzeption keinen besonderen Ausnahmebedingungen, so dass die in dem Anwendungsgebiet bekannten Auswahlkriterien uneingeschränkt Anwendung finden können.

Weitere Einzelheiten, Merkmale und Vorteile des Gegenstandes der Erfindung ergeben sich aus der nachfolgenden Beschreibung der Figuren und der zugehörigen Beispiele sowie Referenzbeispiele.

In den Figuren zeigt:

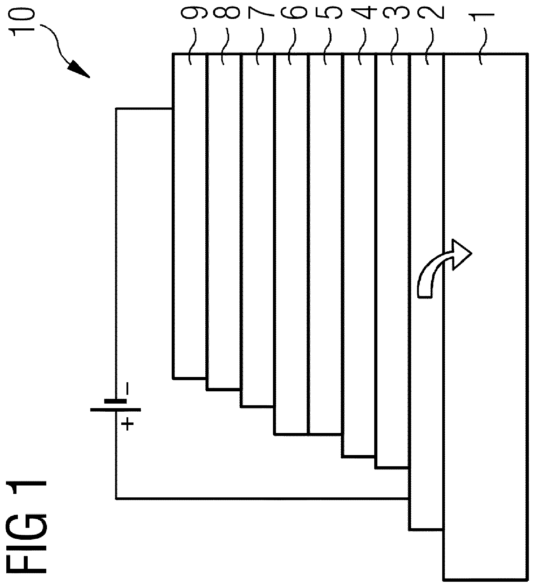

Fig. 1 schematisch den Aufbau einer organischen Leuchtdiode (10). Die Leuchtdiode ist aufgebaut aus einer Glas-Schicht (1); Transparent Conductive oxide (TCO)- oder PEDOT:PPS- oder PANI-Schicht (2); Loch-Injektor-Schicht (3); Loch-Transportschicht (HTL) (4); Emitter-Schicht (EML) (5); Loch-Blocker-Schicht (HBL) (6); Elektronen-Transportschicht (ETL) (7); Elektronen-Injektor-Schicht (8)und einer Kathoden-Schicht (9);

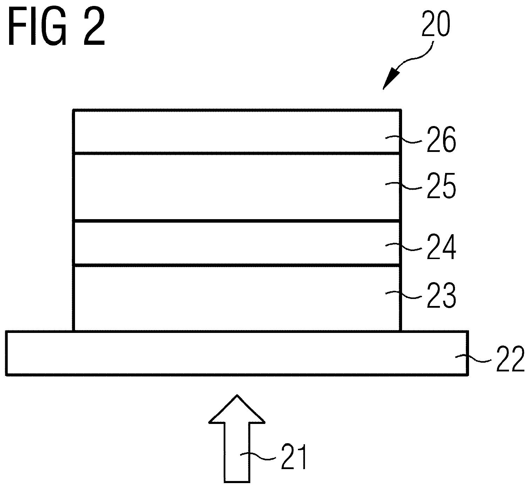

Fig. 2 schematisch den Aufbau einer organischen Solarzelle mit PIN-Struktur (20), welche Licht (21) in elektrischen Strom umwandelt. Die Solarzelle besteht aus einer Schicht aus Indium-Zinn-Oxid (22); einer p-dotierten Schicht(23); einer Absorptions-Schicht (24); einer n-dotierten Schicht (25) und einer Metall-Schicht (26);

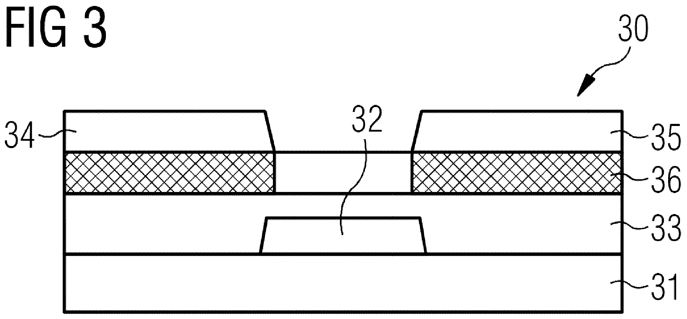

Fig. 3 schematisch einen möglichen Querschnitt eines organischen Feld-Effekt-Transistors (30). Auf einem Substrat (31) ist eine Gate-Elektrode (32), ein Gate-Dielektrikum (33), ein Source und Drain-Kontakt (34 + 35) und ein organischer Halbleiter (36) aufgebracht. Die schraffierten Stellen zeigen die Stellen an denen eine Kontaktdotierung hilfreich ist.

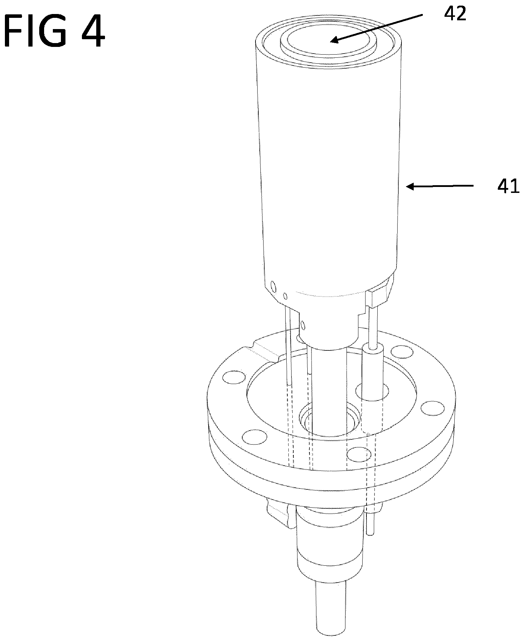

Fig. 4 den Aufbau einer Stand der Technik gemäßen Punktquelle der Firma Creaphys. Die Punktquelle verfügt über einen Tiegel (41). Das abzuscheidende Material wird im Tiegel unter Vakuumbedingungen verdampft. Nach dem Verdampfen des Materials verlassen die Moleküle die Punktquelle über die Austrittsöffnung (42). Aufgrund der hohen mittleren freien Weglänge im Vakuum (10⁻⁵ bis 10⁻⁶ mbar) treffen die Moleküle z.B. eines Metallkomplexes, der als Dotand fungiert, ohne weitere Stöße auf das Substrat. Dies bedeutet, dass ein Material thermisch nur wenig über die Sublimationstemperatur hinaus stabil sein muss, um unzersetzt auf dem Substrat abgeschieden werden zu können. Insbesondere trifft ein mittels einer Punktquelle abgeschiedener Dotierstoff nicht auf Wände der Quelle, sondern kann durch die direkte Anordnung an der Öffnung der Quelle direkt auf das zu beschichtende Substrat abgeschieden werden. Der Bereich des Tiegels, in dem der Dotand verdampft sowie die Austrittsöffnung und das Substrat befinden sich also in einer geradlinigen Anordnung. Insbesondere kann der Dotand abgeschieden werden, ohne über Leitungssysteme oder Sprühsysteme umgeleitet zu werden bevor er auf das Substrat trifft.

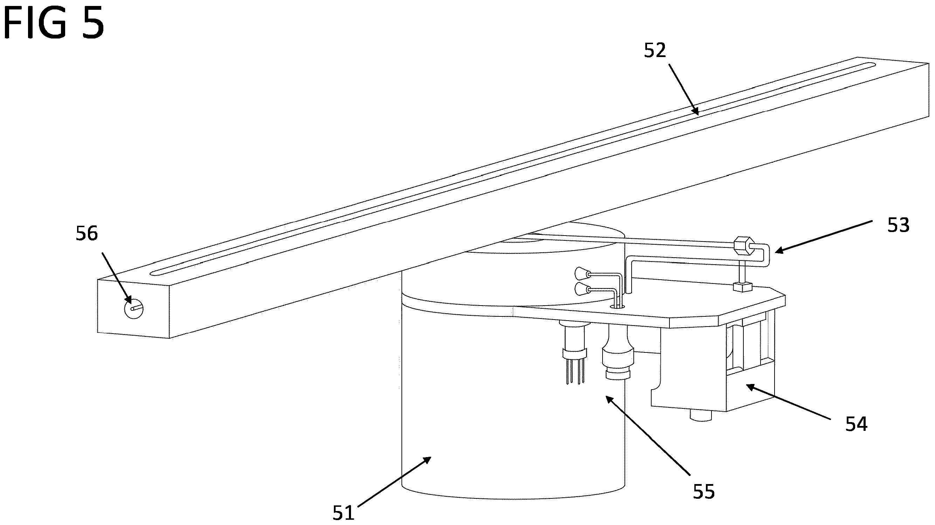

Fig. 5 den Aufbau einer Linearquelle beispielhaft an einer schematisch dargestellten Linearquelle der Firma Vecco. Die Linearquelle verfügt über einen Tiegel (51), der abnehmbar sein kann. Nach dem Verdampfen des abzuscheidenden Materials z.B. des Dotanden im Tiegel, wird der in der Gasphase befindliche Dotand über Leitungen (53) zur Austrittsöffnung geleitet (52). Die Austrittsöffnung (52) kann dabei beispielsweise spaltförmig sein oder aus einer Lochreihe bestehen. Die Linearquelle erlaubt dem Dotanden keinen direkten, geradlinigen Zugang zum Substrat, sondern der Dotand wird vielmehr in der Linearquelle oft mehrmals umgelenkt. Dabei kommt es zu zahlreichen Stößen des Dotanden mit den Wänden der Linearquelle. Die Linearquelle kann ferner steuerbare Ventile (54), Durchflussregler (56) sowie entsprechende Verkabelungen (55) für die elektronische Steuerung etwa der Heizvorrichtung oder der Ventile enthalten. Durch die gezielte Führung des Gasstromes kann eine großflächige Abscheidung besonders gut erreicht werden.

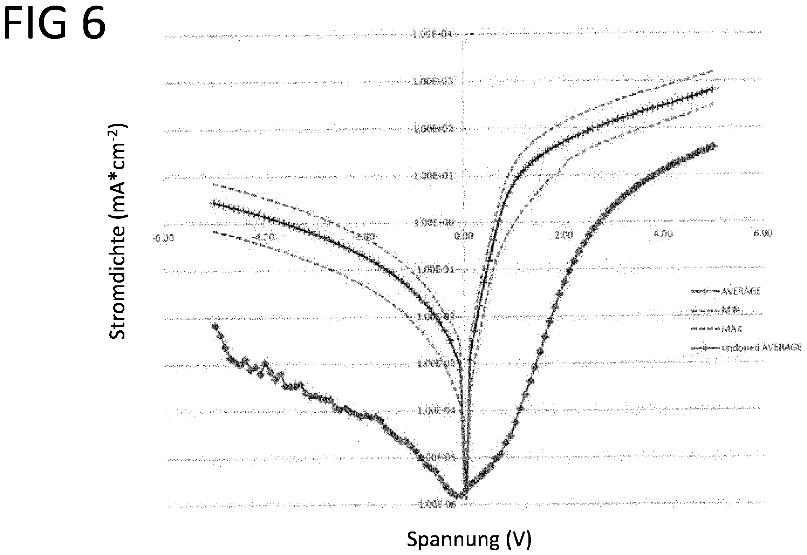

Fig. 6 für Vergleichsbeispiel I die Stromdichte gegen die Spannung für das undotierte Matrixmaterial sowie für das dotierte Matrixmaterial. Als Matrixmaterial wurde hierbei der Lochleiter 2,2',7,7'-tetra(N,N-ditolyl)amino-9,9-spiro-bifluorene, abgekürzt Spiro-TTB, verwendet. Die Stromdichte-Spannungskennlinie demonstriert das ausreichende Dotierverhalten von 15% Cu(3,5-tfmb) in dem Lochleiter Spiro-TTB.

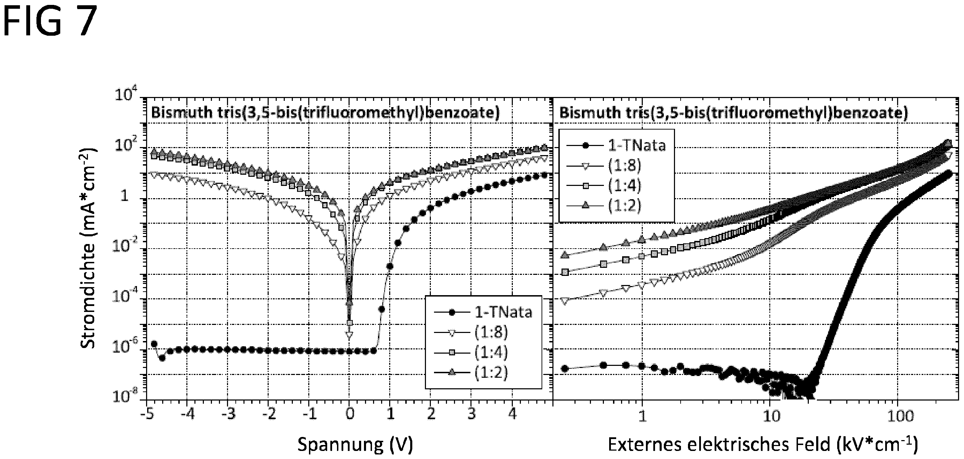

Fig. 7 zeigt im Hinblick auf Beispiel II für mit Bismut(III)-tris-3,5-trifluormethyl-benzoat dotiertes 1-TNata die Stromdichte gegen die Spannung sowie die Stromdichte gegen die externe Feldstärke. Die Messungen wurden an mittels Punktquellen erzeugten dotierten Matrixmaterialien durchgeführt um so einen Vergleich der elektrischen Eigenschaften unter gleichen Bedingungen mit den Vergleichsbeispielen zu ermöglichen. Die Messung wurde bei jeweils drei verschiedenen Dotierstoffgehalten durchgeführt. Die Spannungskennlinie demonstriert gute Leitfähigkeiten und belegt die gute Dotierstoffstärke von Bismut(III)-tris-3,5-trifluormethyl-benzoat.

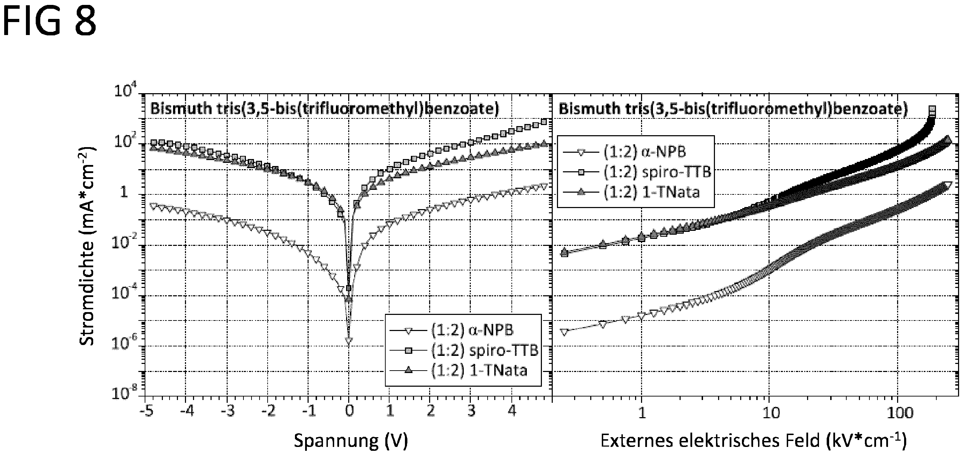

Fig. 8 zeigt im Hinblick auf Beispiel II für die mit Bismut(III)-tris-3,5-trifluormethyl-benzoat dotierten Matrixmaterialien 1-TNata, Spiro-TTB und α-NPB die Stromdichte gegen die Spannung sowie die Stromdichte gegen die externe Feldstärke.

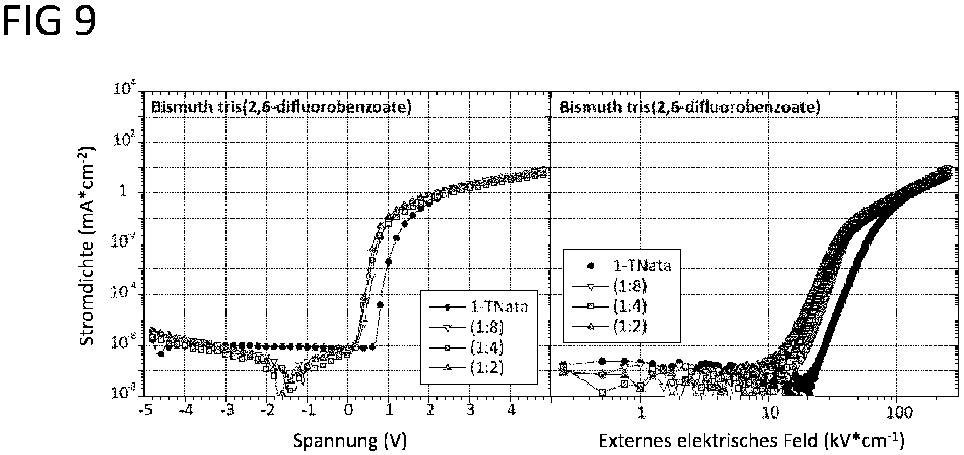

Fig. 9 zeigt im Hinblick auf Referenzbeispiel I für mit Bismut(III)-tris(2,6-difluorobenzoat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

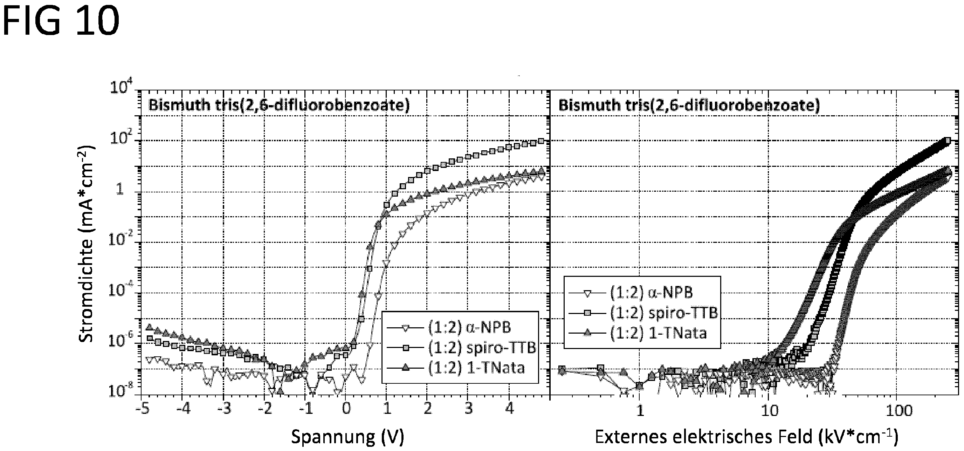

Fig. 10 zeigt im Hinblick auf Referenzbeispiel I für die mit Bismut(III)-tris(2,6-difluorobenzoat) dotierten Matrixmaterialien 1-TNata, Spiro-TTB und α-NPB die Stromdichte gegen die Spannung sowie die Stromdichte gegen die externe Feldstärke.

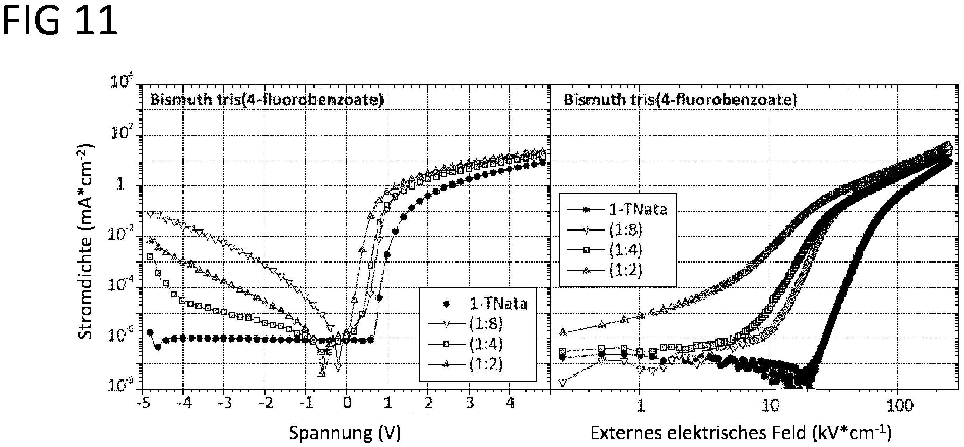

Fig. 11 zeigt im Hinblick auf Referenzbeispiel II für mit Bismut(III)-tris(4-fluorobenzoat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

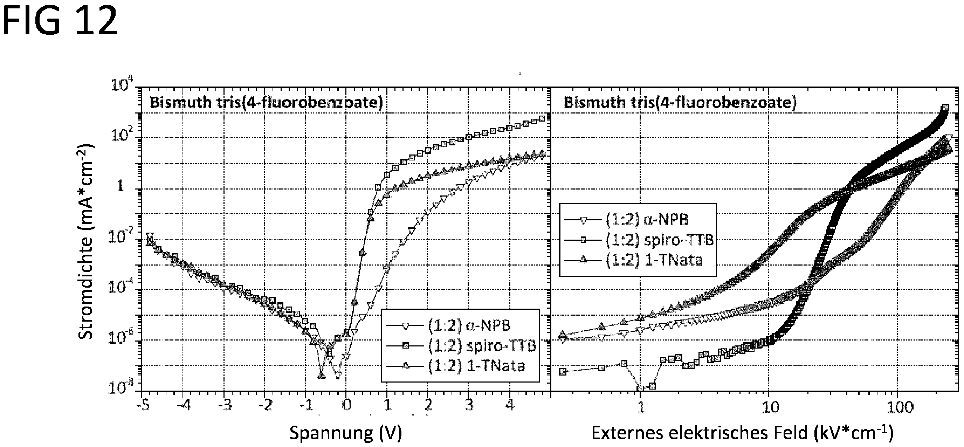

Fig. 12 zeigt im Hinblick auf Referenzbeispiel II für die mit Bismut(III)-tris(4-fluorobenzoat) dotierten Matrixmaterialien 1-TNata, Spiro-TTB und α-NPB die Stromdichte gegen die Spannung sowie die Stromdichte gegen die externe Feldstärke.

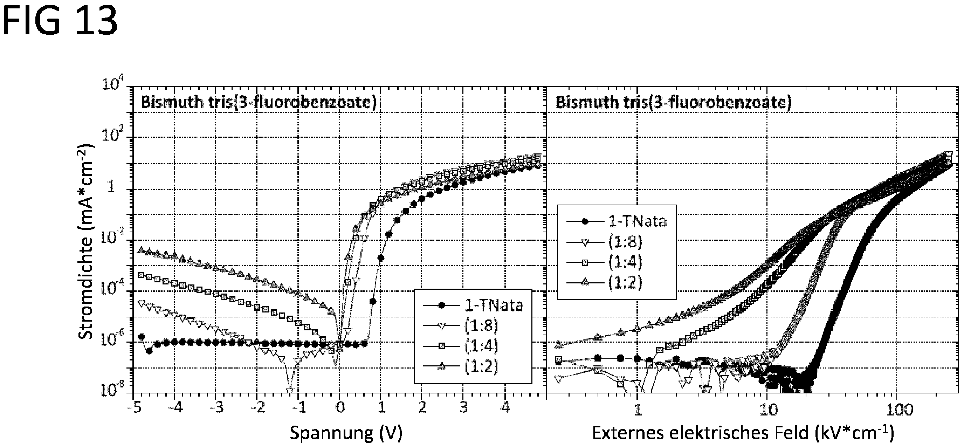

Fig. 13 zeigt im Hinblick auf Referenzbeispiel III für mit Bismut(III)-tris(3-fluorobenzoat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

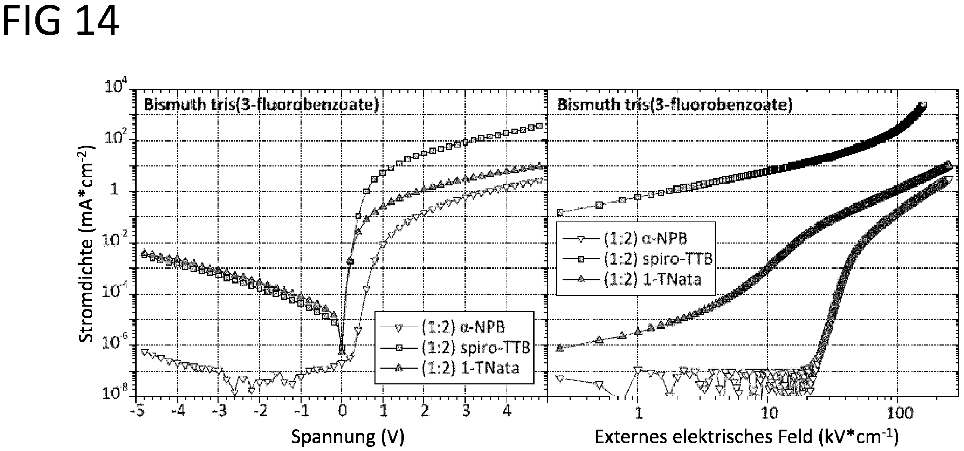

Fig. 14 zeigt im Hinblick auf Referenzbeispiel III für die mit Bismut(III)-tris(3-fluorobenzoat) dotierten Matrixmaterialien 1-TNata, Spiro-TTB und α-NPB die Stromdichte gegen die Spannung sowie die Stromdichte gegen die externe Feldstärke.

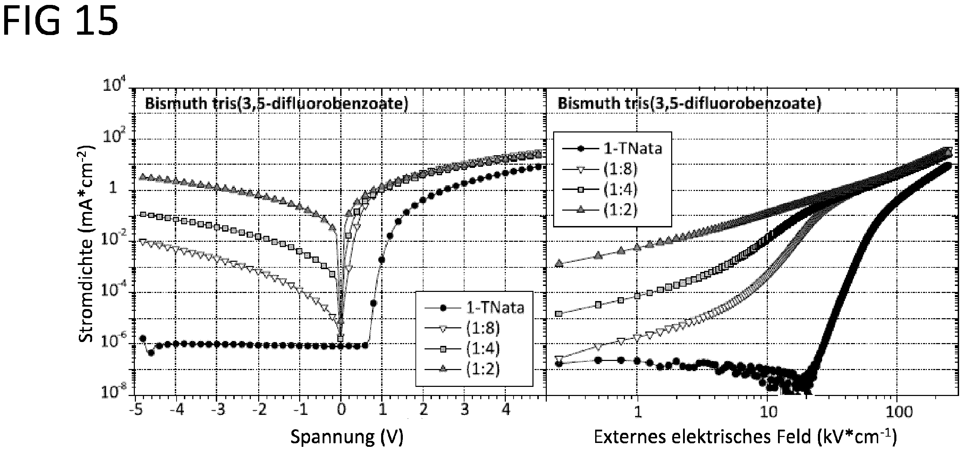

Fig. 15 zeigt im Hinblick auf Referenzbeispiel IV für mit Bismut(III)-tris(3,5-difluorobenzoat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

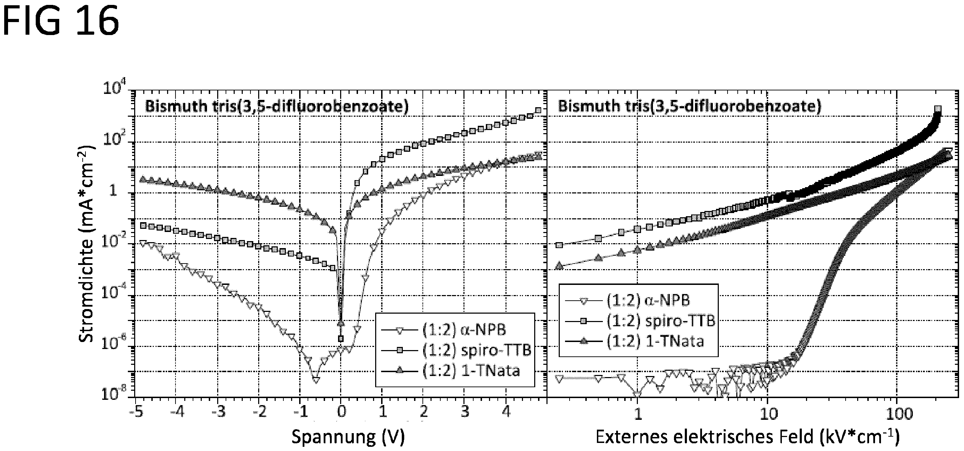

Fig. 16 zeigt im Hinblick auf Referenzbeispiel IV für die mit Bismut(III)-tris(3,5-difluorobenzoat) dotierten Matrixmaterialien 1-TNata, Spiro-TTB und α-NPB die Stromdichte gegen die Spannung sowie die Stromdichte gegen die externe Feldstärke.

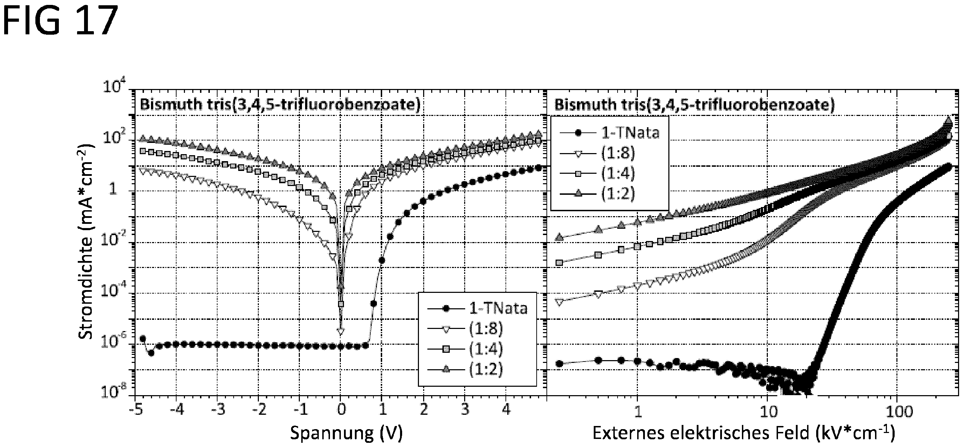

Fig. 17 zeigt im Hinblick auf Referenzbeispiel V für mit Bismut(III)-tris(3,4,5-trifluorobenzoat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

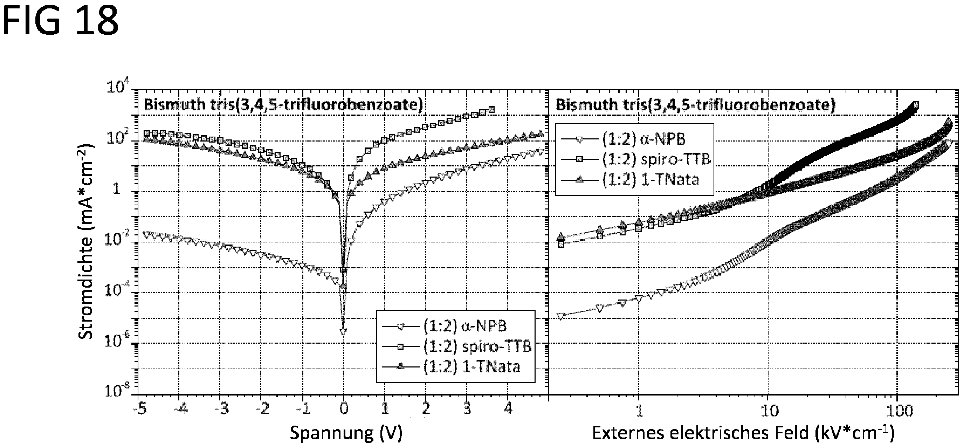

Fig. 18 zeigt im Hinblick auf Referenzbeispiel V für die mit Bismut(III)-tris(3,4,5-trifluorobenzoat) dotierten Matrixmaterialien 1-TNata, Spiro-TTB und α-NPB die Stromdichte gegen die Spannung sowie die Stromdichte gegen die externe Feldstärke.

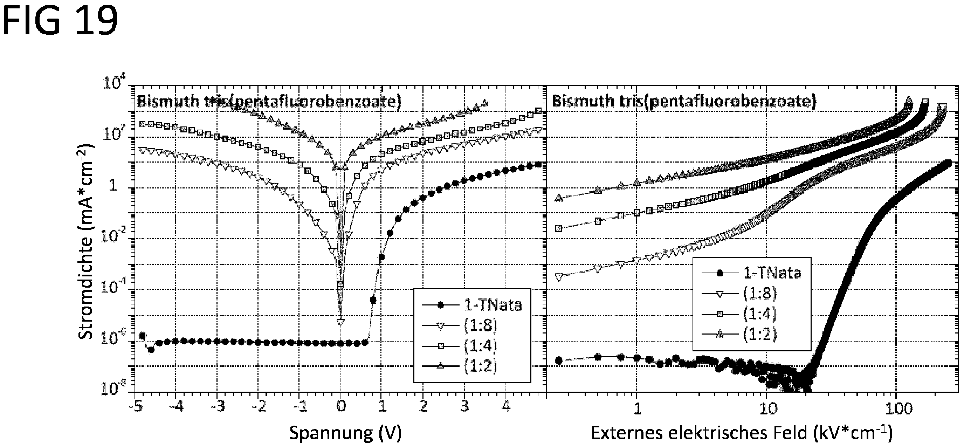

Fig. 19 zeigt im Hinblick auf Referenzbeispiel VI für mit Bismut(III)-tris(perfluorobenzoat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

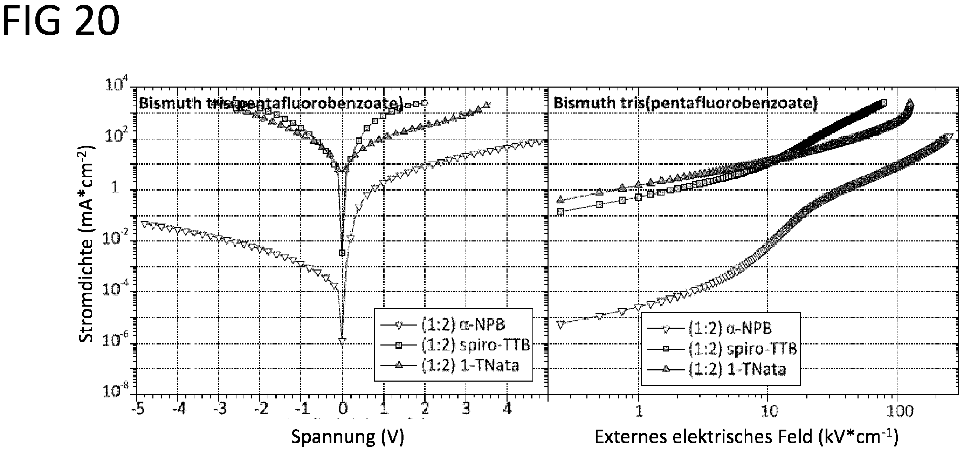

Fig. 20 zeigt im Hinblick auf Referenzbeispiel VI für die mit Bismut(III)-tris(perfluorobenzoat) dotierten Matrixmaterialien 1-TNata, Spiro-TTB und α-NPB die Stromdichte gegen die Spannung sowie die Stromdichte gegen die externe Feldstärke.

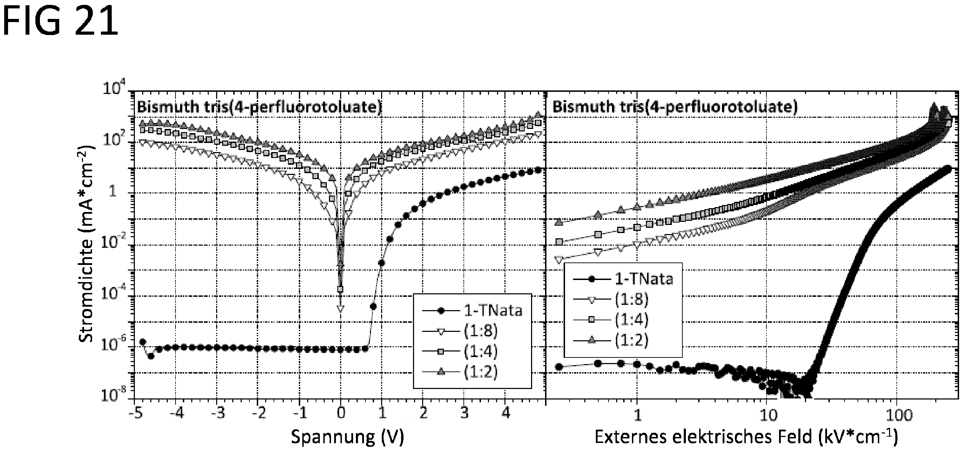

Fig. 21 zeigt im Hinblick auf Referenzbeispiel VII für mit Bismut(III)-tris(4-perfluorotoluat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

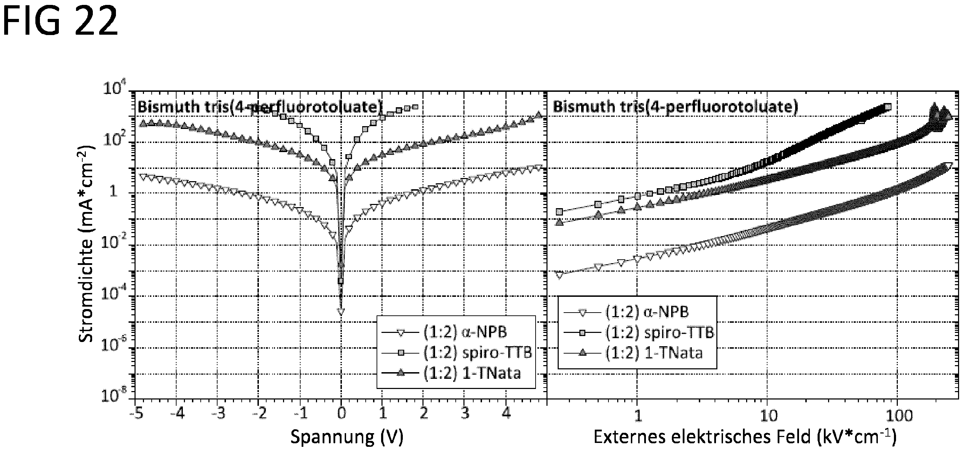

Fig. 22 zeigt im Hinblick auf Referenzbeispiel VII für die mit Bismut(III)-tris(4-perfluorotoluat) dotierten Matrixmaterialien 1-TNata, Spiro-TTB und α-NPB die Stromdichte gegen die Spannung sowie die Stromdichte gegen die externe Feldstärke.

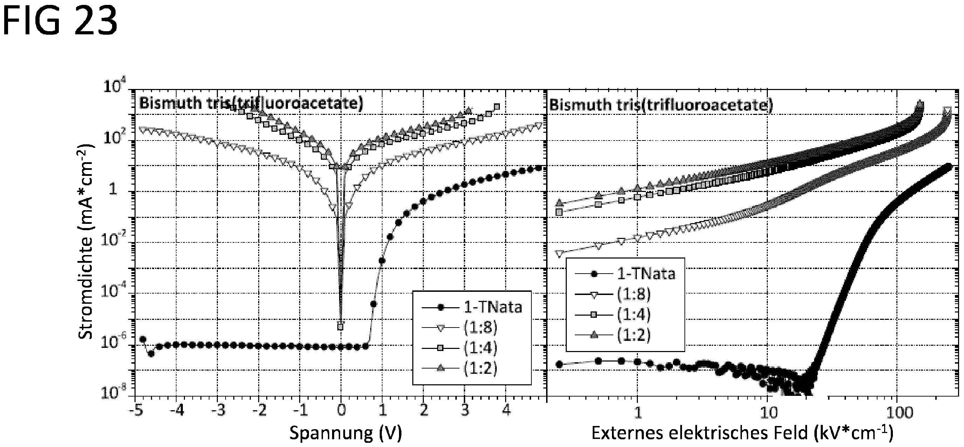

Fig. 23 zeigt im Hinblick auf Referenzbeispiel VIII für mit Bismut(III)-tris(trifluoracetat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

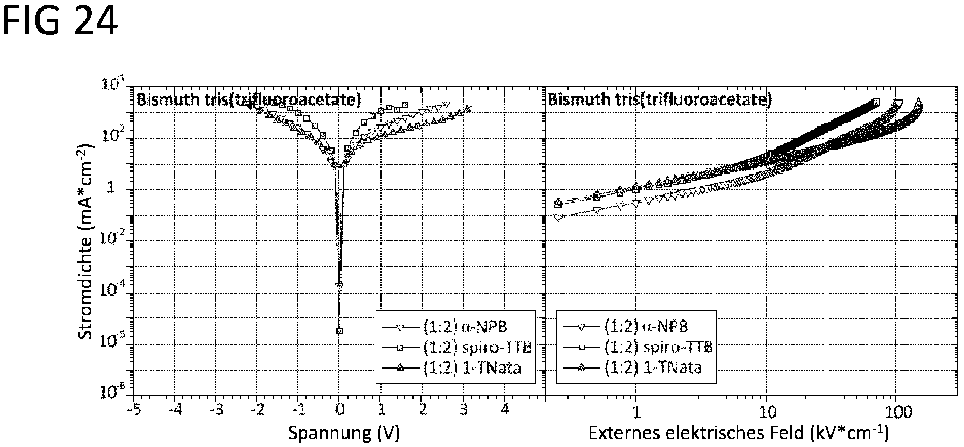

Fig. 24 zeigt im Hinblick auf Referenzbeispiel VIII für die mit Bismut(III)-tris(trifluoracetat) dotierten Matrixmaterialien 1-TNata, Spiro-TTB und α-NPB die Stromdichte gegen die Spannung sowie die Stromdichte gegen die externe Feldstärke.

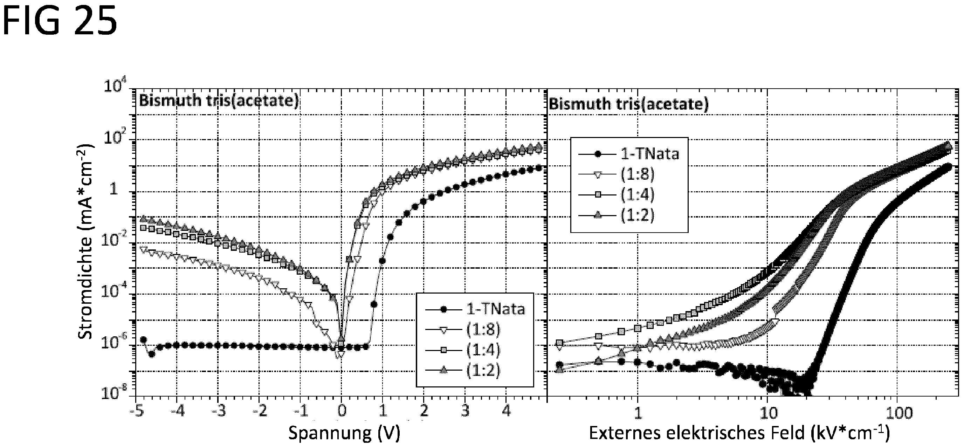

Fig. 25 zeigt im Hinblick auf Referenzbeispiel IX für mit Bismut(III)-tris(triacetat) dotiertes 1-TNata die Stromdichte gegen die Spannung sowie die Stromdichte gegen die externe Feldstärke für das durch Abscheidung mittels Punktquellen erhaltene dotierte Matrixmaterial. Die Messung wurde bei jeweils drei verschiedenen Dotierstoffgehalten durchgeführt.

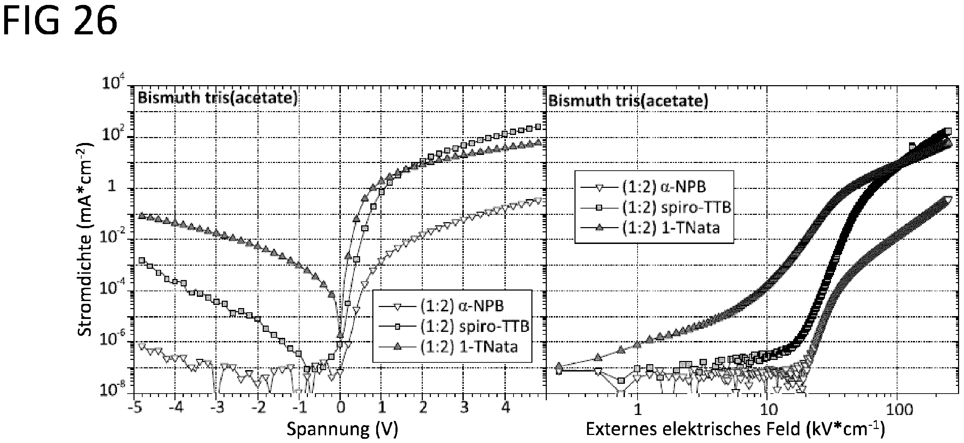

Fig. 26 zeigt im Hinblick auf Referenzbeispiel IX für die mit Bismut(III)-tris(triacetat) dotierten Matrixmaterialien 1-TNata, Spiro-TTB und α-NPB die Stromdichte gegen die Spannung sowie die Stromdichte gegen die externe Feldstärke.
Im Folgenden sollen Vergleichsbeispiele I und Beispiel II vorgestellt werden. Die beiden Metallkomplexe Kupfer(I)bis-trifluor-methylbenzoat (Vergleichsbeispiel I) und Bismut(III)-tris-3,5-trifluormethylbenzoat (Beispiel II) sind außerordentlich Temperaturstabil mit Zersetzungstemperaturen deutlich über ihrer Sublimationstemperatur. Beide Komplexe können mittels Quellen abgeschieden werden, in welchen die Komplexe Stöße mit zumindest einer Wand der Quelle erfahren. Beispielsweise besitzen beide Komplexe eine hinreichend hohe Stabilität für eine Gasphasenabscheidung über Linearquellen. Dies wurde durch sogenannte Ampullen-Tests experimentell bestätigt.

Alle weiteren Referenzbeispiele, nämlich die Referenzbeispiele I bis IX, zeigten in Ampullen-Tests keine ausreichende Stabilität. Die Erfinder haben experimentell festgestellt, dass diese Substanzen nicht für eine Abscheidung mittels Quellen geeignet sind, in welchen die Komplexe Stöße erfahren.

Die elektrischen Eigenschaften der jeweiligen dotierten Schichten werden im Folgenden ebenfalls untersucht. Da die Metallkomplexe der Referenzbeispiele I bis IX jeweils nicht hinreichend stabil für eine Abscheidung mittels Quellen sind, bei welchen es zu Stößen mit zumindest einer Wand der Quelle kommt, wurden Messungen an über Punktquellen abgeschiedenen organischen elektrischen Schichten durchgeführt und miteinander verglichen. Beispielsweise sind die Komplexe der Referenzbeispiele I bis IX nicht stabil genug für eine Abscheidung mittels Linearquellen.

### Vergleichsbeispiel I

Vergleichsbeispiel I bezieht sich auf den Metallkomplex Kupfer(I)bis-trifluormethylbenzoat - im Folgenden abgekürzt mit Cu(3,5-tfmb) - handelt.

Allgemein lassen sich viele Kupfer(I)-Komplexe fluorierter Derivate von Benzoaten auf folgendem Weg herstellen:

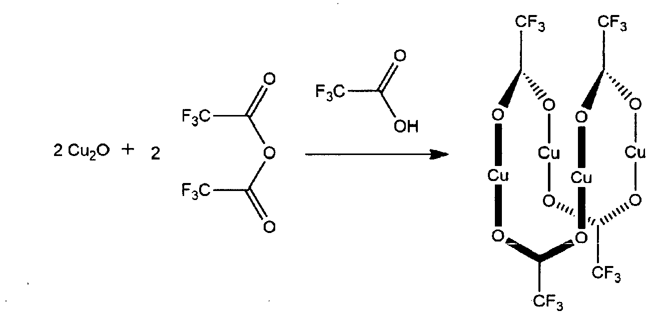

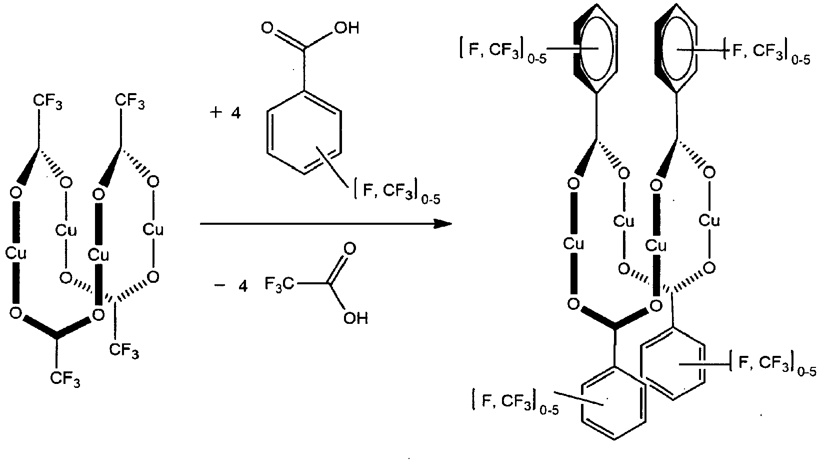

Auf analoge Weise können eine Vielzahl der im beschriebenen Verfahren zum Einsatz kommenden Metallkomplexe hergestellt werden.

So kann etwa der Metallkomplex 3,5-Bis-(trifluormethyl-benzoat) aus Cu(I)-trifluoracetat gemäß folgender Vorschrift erhalten werden:
5 g (7,08 mmol) Cu(I)-trifluoracetat wird zusammen mit 7,5 g (29,03 mmol) 3,5-Bis-(trifluormethylbenzoesäure) in einem 250 ml Zweihalskolben unter Inertgas (beispielsweise in einer Glovebox) eingewogen. Die Mischung wird mit 80 ml Toluol und 70 ml Benzol versetzt wobei eine grünliche Reaktionslösung entsteht. Diese wird über Nacht unter leichtem Rückfluss erhitzt (Badtemperatur ca. 90°C) woraufhin das Lösungsmittel abdestilliert wird. Es bleibt ein cremefarben-gräuliches Produkt, das im Vakuum getrocknet wird. Die Ausbeute beträgt 6,98 g (76%) nach einmaliger Sublimation. Der Sublimationsbereich der Substanz liegt bei 160-180°C bei 2 x 10⁻⁵ mbar.

Durch Ampullen-Tests, konnte nachgewiesen werden, dass Cu(3,5-tfmb) bis mindestens 225°C stabil ist.

Bei Ampullen-Tests werden bei einem Basisdruck von 10⁻⁵ bis 10⁻⁶ mbar, ca. 100 bis 500 mg der zu untersuchenden Substanz eingeschmolzen. Anschließend wird die Ampulle im Ofen erhitzt und bei der jeweiligen Temperatur für ca. 100 Stunden belassen. Durch eine optische Prüfung kann erkannt werden, ob es zu einer Zersetzung des Metallkomplexes gekommen ist, da eine Zersetzung zu einer Verfärbung, häufig zu einer Braunfärbung führt. Die Ampulle wird schließlich nach etwa 100 Stunden bei der ersten Testtemperatur in 10-20 Kevin Schritten weiter erhitzt und wiederum für etwa 100 Stunden bei der neuen Testtemperatur im Ofen belassen. Das Experiment wird fortgeführt, bis schließlich eine Verfärbung auf Zersetzung schließen lässt.

In einem Kontrollexperiment wird zusätzlich jeweils im Anschluss an die optische Bestimmung eine weitere Ampulle mit einer neuen Probe des gleichen zu testenden Metallkomplexes wiederum im Ofen für etwa 100 Stunden erhitzt. Das Erhitzten erfolgt dabei auf eine Temperatur knapp unter der optisch ermittelten Zersetzungstemperatur. Die so behandelte Probe wird im Anschluss mittels Elementaranalyse untersucht und so über die Elementarzusammensetzung die Stabilität des Komplexes bestätigt.

Für Kupfer(I)bis-trifluormethylbenzoat gemäß Vergleichsbeispiel I wurden Ampullen-Tests für drei verschiedene Temperaturen durchgeführt: 210°C, 230°C und 240°C.

Die Messungen belegen, dass der Komplex bis mindestens 225°C stabil ist. Dies wurde mittels Elementaranalyse bestätigt.

Cu(3,5-tfmb) ist damit für die Abscheidung aus der Gasphase mittels auch aus Quellen geeignet, bei welchen es zu Stößen des Komplexes mit zumindest einer der Wände der Quellen kommt. Beispielsweise ist eine Abscheidung aus der Gasphase über Linearquellen möglich.

Mit Cu(3,5-tfmb) dotierte Schichten besitzen eine sehr gute optische Transparenz im sichtbaren Bereich. Cu(3,5-tfmb) zeichnet sich zudem durch eine ausreichend gute Dotierstoffstärke aus.

Wie ferner durch Figur 6 gezeigt verfügen mit Cu(3,5-tfmb) dotierte organische Schichten über gute Leitfähigkeiten.

### Beispiel II

Beispiel II bezieht sich auf ein beschriebenes Verfahren, wobei es sich bei dem Metallkomplex um Bismut(III)-tris-3,5-trifluormethylbenzoat - im Folgenden abgekürzt mit Bi(3,5-tfmb)₃ - handelt.

Bismut-Komplexe gemäß Beispiel II sowie die im Folgenden beschriebenen Metallkomplexe der Referenzspielen I bis VII wurden nach folgendem allgemeinen Verfahren gemäß Schema 1 hergestellt:

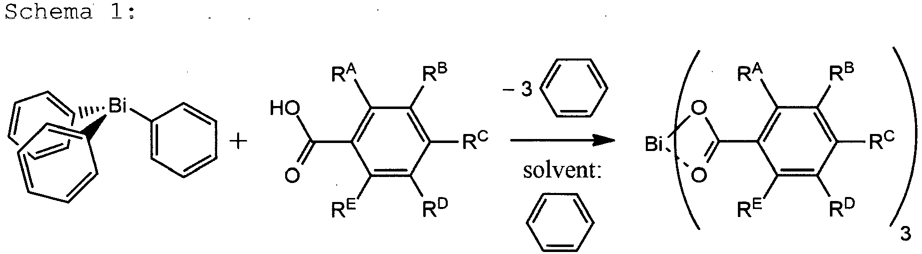

Für Bi(3,5-tfmb)₃ sind dabei die Reste R^{B} und R^{D}, also die Reste in 3,5-Position, jeweils CF₃-Substituenten und die Reste R^{A}, R^{C} und R^{E} jeweils Wasserstoffatome.

Der Erhalt von Bi(3,5-tfmb)₃ wurde nach Reinigung mittels Sublimation durch Elementaranalyse bestätigt (gemessen: Kohlenstoff in % 33,5; Wasserstoff in % 0,5; berechnet: Kohlenstoff in % 33,06; Wasserstoff in % 0,92).

Die thermische Stabilität von Bismut(III)-tris-3,5-trifluormethylbenzoat wurde mit Hilfe von Ampullen-Tests, wie sie bereits in Zusammenhang mit Vergleichsbeispiel I beschrieben wurden bestimmt. Der Metallkomplex ist demnach auch bei thermischer Behandlung über eine Zeitspanne von 144 Stunden bei 330°C stabil. Bei Temperaturen unterhalb von 330°C sind keine Verfärbungen der Ampulle zu beobachten. Erst ab 330°C ist eine leichte Verfärbung zu sehen. Auch Daten der Elementaranalyse bestätigen, dass der Komplex im Rahmen des statistischen Fehlers bis 330°C thermisch stabil ist.

Wie in Tabelle 1 gezeigt wurden zudem analoge Tests jeweils für 144 Stunden bei den Temperaturen 260°C, 280°C, 315°C und 330°C durchgeführt. Es wurden dabei Proben der temperaturbehandelten Substanz jeweils mittels Elementaranalyse untersucht, wobei der Kohlenstoffgehalt bestimmt wurde. Anhand der Abweichung des so ermittelten Kohlenstoffgehalts vom zu erwartenden Kohlenstoffgehalt des unzersetzten Komplexes sind so Rückschlüsse über den Zersetzungsgrad des Komplexes nach der jeweiligen Temperaturbehandlung möglich. Durch die Testreihe konnte von den Erfindern belegt werden, dass die Bismut(III)-tris-3,5-trifluormethylbenzoat gemäß Beispiel II eine besonders hohe thermische Stabilität aufweisen und unter Berücksichtigung des statistischen Fehlers erst bei Temperaturen oberhalb von 330°C einen deutlichen Zerfall zeigen.

**Tabelle 1: Bestimmung des Kohlenstoffgehaltes mittels Elementaranalyse an zwei verschiedenen Orten (Ort A, Ort B) der Ampulle an welchen sich nach dem Ampullen-Test jeweils Substanz abgeschieden hat.**

| Die geringen Abweichungen des ermittelten Kohlenstoffgehalts bis zu Temperaturen von 330°C vom theoretisch berechneten Gehalt (von 33,06%) belegen die hohe thermische Stabilität von Bismut(III)-tris-3,5-trifluormethylbenzoat. Erst bei Temperaturen über 330°C wird eine deutliche Zersetzung des Komplexes beobachtet. | | |
|---|---|---|
| | Ort A | Ort B |
| 3x sublimiertes Material | kein Material | theoret. Wert + 0.44% C |
| 144h bei 260°C | theoret. Wert + 0.49% C | theoret. Wert + 0.05% C |
| 144h bei 280°C | theoret. Wert + 0.63% C | theoret. Wert + 0.12% C |
| 144h bei 300°C | theoret. Wert + 0.49% C | kein Material |
| 144h bei 315°C | theoret. Wert + 0.47% C | theoret. Wert + 0.18% C |
| 144h bei 330°C | theoret. Wert + 0.14% C | Zersetzung |

Die Elementaranalyse bestätigt damit eine Stabilität des Komplexes unter Berücksichtigung des statistischen Fehlers bis zu 330°C.

Aufgrund der hohen thermischen Stabilität sind damit Dotierstoffe mit fluorierten Alkylsubstituenten R¹, wie am Beispiel von Bismut(III)-tris-3,5-trifluormethylbenzoat zu sehen ist, besonders für eine Gasphasenabscheidung mittels Quellen geeignet, bei welchem die Metallkomplexe also die Dotanden Stöße mit zumindest einer Wand der Quelle erfahren. Beispielsweise sind die Metallkomplexe stabil genug um ohne Zersetzung über Linearquellen aus der Gasphase abgeschieden werden zu können.

Mit Bi(3,5-tfmb)₃ dotierte Schichten besitzen eine sehr gute optische Transparenz im sichtbaren Bereich.

Bi(3,5-tfmb)₃ zeichnet sich zudem durch eine ausreichend gute Dotierstoffstärke aus. Dies wird durch die im Folgenden zusammengefassten experimentellen Daten weiter verdeutlicht.

Die elektrischen Eigenschaften von mit Bi(3,5-tfmb)₃ dotierten organischen Schichten werden in den Figuren 7, 8 und den Tabellen 2, 3 zusammengefasst.

**Tabelle 2: Zusammenfassung der elektrischen Eigenschaften von mit Bi(3,5-tfmb)₃ dotiertem 1-TNata. Es werden insbesondere die elektrischen Eigenschaften bei drei verschiedenen Dotierstoffgehalten an der Matrix untersucht (1:8, 1:4 und 1:2).**

| | *(1:8)* | *(1:4)* | *(1:2)* |
|---|---|---|---|
| *Exp. molar ratio* | 1/8.08 (7.64 _{vol.}%) | 1/4.07 (14.11 _{vol.}%) | 1/1.99 (25.14 _{vol.}%) |
| *σ₀ (S·cm⁻¹)* | 4.08·10⁻⁷ (± 1.00 %) | 7.01·10⁻⁷ (±4.61 %) | 5.27·10⁻⁷ (± 0.88 %) |
| *ρ_{c.;0} (Ω·cm⁻²)* | 1.96·10⁷ (± 5.65 %) | 1.72·10⁶ (± 5.14 %) | 4.64·10⁵ (± 3.86 %) |
| *E_{bi} (kV·cm⁻¹)* | < 0.25 | | |
| *εᵣ* | Too conductive | | |
| *r* + *1* | 3.24 (± 1.72 %) | 2.33 (± 4.61 %) | 1.59 (± 2.42 %) |
| *µ₀ (cm²·V⁻¹·s⁻¹)* | 3.20·10⁻⁵ (± 25 %)* | Ballistic | |
| *γ (cm*^{*1*/*2*}·*V*^{*-1*/}*²)* | 2.54·10⁻⁴ (± 4.9 %)* | | |

Die in Tabelle 2 sowie allen weiteren Tabellen für die verschiedenen Materialien angeführten elektrischen Eigenschaften wurden durch Messungen an auf ITO-Substraten (Indium-Tin-oxide) geträgerten, organischen 200 nm dicken Schichten durchgeführt, die durch Koverdampfung über Punktquellen erhalten wurden.

Exp. molar ratio steht hierbei jeweils für das molare Verhältnis von Matrixmaterial und dem Metallkomplex. σ₀ steht für die Leitfähigkeit der gemessenen organischen elektronischen Schicht. ρ_{c.;0} steht für den Kontaktwiderstand. E_{bi} steht für die elektrische Feldstärke des inneren elektrischen Feldes des Halbleitermaterials (englischsprachiger Fachbegriff "built-in electric field"; Diese Feldstärke ergibt sich aus der Differenz der Austrittsarbeit zwischen Anode und Kathode des organischen elektronischen Bauteils). εᵣ gibt die Dielektrizitätskonstante des durch Koverdampfung erhaltenen Materials an.

Eine Reihe von weiteren Parametern wurde bestimmt im Zusammenhang mit dem Transport-Regime der Ladungsträger in der organischen elektrischen Schicht, wie sie in verschiedenen Leitfähigkeitstheorien in der Literatur beschrieben sind. r steht hierbei für einen empirischen Faktor (englisch-sprachiger Fachbegriff "trap distribution factor"), der eine exponentielle Verteilung gemäß den Ladungsträger-Transportmodellen beschreibt (Steiger et al. "Energetic trap distributions in organic semiconductors" Synthetic Metals 2002, 129 (1), 1-7; Schwoerer et al. "Organic Molecular Solids", Wiley-VCH, 2007). µ₀ steht für die Ladungsträgermobilität und γ steht für den Feldaktivierungsfaktor (englischsprachiger Fachbegriff: "field-activation factor") an. γ ist etwa von Bedeutung im Zusammenhang mit einer Beschreibung des Ladungstransports gemäß der Murgatroyd-Gleichung: Murgatroyd, P. N. "Theory of space-charge-limited current enhanced by Frenkel effect" Journal of Physics D: Applied Physics 1979, 3 (2), 151.

Die in Tabelle 2 bis 31 genutzten Begriffe "too conductive", "ballistic", "no ohmic contact", "trapping", "aging", "no TFLC", "Compliance" haben dabei jeweils folgende Bedeutung: Der Begriff "too conductive" beschreibt, dass die Messung aufgrund einer zu hohen Leitfähgikeit der Schicht nicht aussagekräftig ist. Der Begriff "no ohmic contact" besagt, dass kein elektrischer Kontakt vorlag. Der Begriff "Compliance" bringt zum Ausdruck, dass die voreingestellte Strombegrenzung des Messgerätes erreicht wurde. Der Begriff "TFLC" steht für "trap-filled limited regime" gemäß den genannten Arbeiten von Steiger *et al.* und Schwoerer *et al.* und verweist auf ein Transport-Regime für die Ladungsträger der organischen elektrischen Schicht. Die Begriffe "ballistic", "trapping" und "aging" stehen für "ballistisch", "einfangend" und "alternd". Die Fachbegriffe "ballistic", "trapping" und "aging" beziehen sich dabei auf weitere Transport-Regime gemäß der unterschiedlichen in der Literatur beschriebenen Leitfähigkeitsmodelle. Die unterschiedlichen Leitfähigkeits-regime können hierbei am Exponenten der Strom-Spannungsabhängigkeit erkannt werden.

Die jeweiligen Abkürzungen gelten in analoger Weise auch für die Tabellen 3 bis 21.

**Tabelle 3: Zusammenfassung der elektrischen Eigenschaften von mit (1:2) Bi(3,5-tfmb)₃ dotierten Matrixmaterialien 1-TNata, α-NPB und spiro-TTB. Die elektrischen Eigenschaften bei Dotierung verschiedener Matrixmaterialien werden verglichen.**

| | *1-TNata* | *α-NPB* | *spiro-TTB* |
|---|---|---|---|
| *Exp. molar ratio* | 1/1.99 (25.14 _{vol.}%) | 1/1.99 (35.32 _{vol.}%) | 1/2.00 (19.99 _{vol.}%) |
| *σ₀ (S·cm⁻¹)* | 5.27·10⁻⁷ (± 0.88 %) | 1.05·10⁻⁷ (± 1.23 %) | 4.60·10⁻⁶ (± 0.63 %) |
| *ρ_{c.;0} (Ω*·*cm⁻²)* | 4.64·10⁵ (± 3.86 %) | 3.55·10⁸ (± 7.18 %) | 4.76·10⁵ (± 4.76 %) |
| *E_{bi} (kV*·*cm⁻¹)* | < 0.25 | | |
| *εᵣ* | Too conductive | 2.15 (± 5.07 %) | Too conductive |
| *r* + *1* | 1.59 (± 2.42 %) | 3.15 (± 3.71 %) | 2.24 (± 3.01 %) |
| *µ₀ (cm²*·*V⁻¹*·*s⁻¹)* | Ballistic | 5.28·10⁻⁵ (± 37.3 %) | Ballistic |
| *γ (cm*^{*1*/}*²·V*^{*-1*/}*²)* | | 1.88·10⁻⁶ (± 11.4 %) | |

Die Messungen bestätigen, dass mit Bi(3,5-tfmb)₃ dotierte Matrixmaterialien über gute elektrische Eigenschaften, insbesondere ausreichend gute Leitfähigkeiten verfügen.

Dies wird im Folgenden durch einen Vergleich mit den elektrischen Eigenschaften einer Vielzahl weiterer Komplexe verdeutlicht, welche im Gegensatz zu Cu(3,5-tfmb) gemäß Vergleichsbeispiel I und Bi(3,5-tfmb)₃ gemäß Beispiel II in Ampullen-Tests jedoch jeweils keine ausreichende Temperaturbeständigkeit zeigten und daher nicht für eine Abscheidung aus der Gasphase mittels Quellen geeignet sind, bei welchen Stöße mit zumindest einer Wand der Quelle erfolgen.

### Referenzbeispiel I

Referenzbeispiel I betrifft die Verwendung von Bismut(III)-tris(2,6-difluorobenzoat), kurz Bi(2,6-dfb)₃, als Metallkomplex für eine Gasphasenabscheidung.

Die Synthese von Bi(2,6-dfb)₃ wurde gemäß Schema 1 durchgeführt. Für Bi(2,6-dfb)₃ sind dabei in Schema 1 die Reste R^{A} und R^{E} jeweils Fluoratome und die restlichen Substituenten R^{B}, R^{C} und R^{D} jeweils Wasserstoffatome.

Der Erhalt von Bi(2,6-dfb)₃ wurde nach Reinigung mittels Sublimation durch Elementaranalyse bestätigt (gemessen: Kohlenstoff in % 36,2; Wasserstoff in % 1,5; berechnet: Kohlenstoff in % 37,06; Wasserstoff in % 1,32).

Die elektrischen Eigenschaften von mit Bi(2,6-dfb)₃ dotierten organischen Schichten werden in den Figuren 9, 10 und den Tabellen 4, 5 zusammengefasst.

**Tabelle 4: Zusammenfassung der elektrischen Eigenschaften von mit Bi(2,6-dfb)₃ dotiertem 1-TNata.**

| | *(1:8)* | *(1:4)* | *(1:2)* |
|---|---|---|---|
| *Exp. molar ratio* | 1/8.07 (4.57 ᵥₒₗ%) | 1/3.96 (8.90 _{vol.}%) | 1/2.00 (16.22 _{vol.}%) |
| *σ₀ (S·cm⁻¹)* | 6.98·10⁻⁸ (± 2.18 %) | 3.62·10⁻⁸ (± 2.56 %) | 4.80·10⁻⁸ (± 1.25 %) |
| *ρ_{c.;0} (Ω*·*cm⁻²)* | no ohmic contact | | |
| *E_{bi} (kV*·*cm⁻¹)* | 13.6 (± 19.3 %) | 12.3 (± 12.2 %) | 9.50 (± 13.2 %) |
| *εᵣ* | 2.48 (± 4.21 %) | 2.45 (± 11.9 %) | 3.51 (± 53.2 %) |
| *r* + *1* | 15.2 (± 6.60 %) | 13.3 (± 6.80 %) | 12.1 (± 6.11 %) |
| *µ₀ (cm²*·*V⁻¹*·*s⁻¹)* | 4.82·10⁻⁶ (± 8.71 %) | 3.18·10⁻⁶ (± 16.6 %) | 3.95·10⁻⁶ (± 58.2 %) |
| *γ (cm*^{*1*/*2*}·*V*^{*-1*/}*²)* | 2.01·10⁻³ (± 0.81 %) | 1.78·10⁻³ (± 0.89 %) | 9.82·10⁻⁴ (± 1.23 %) |

**Tabelle 5: Zusammenfassung der elektrischen Eigenschaften von mit (1:2) Bi (2,6-dfb)₃-dotierten Matrixmaterialien 1-TNata, α-NPB und spiro-TTB.**

| | *I-TNata* | *α-NPB* | *spiro-TTB* |
|---|---|---|---|
| *Exp. molar ratio* | 1/2.00 (16.22 _{vol.}%) | 1/2.02 (23.72 _{vol.}%) | 1/1.96 (12.87 _{vol.}%) |
| *σ₀ (S*·*cm*⁻*¹)* | 4.80·10⁻⁸ (± 1.25 %) | 4.89·10⁻⁹ (± 2.82 %) | 2.04·10⁻⁷ (± 1.90 %) |
| *ρ_{c.;0} (Ω*·*cm⁻²)* | no ohmic contact | | |
| *E_{bi} (kV*·c*m⁻¹)* | 9.50 (± 13.2 %) | 30.0 (± 9.17 %) | 12.5 (± 46.0 %) |
| *εᵣ* | 3.51 (± 53.2 %) | 2.34 (± 3.83 %) | 3.01 (± 5.83 %) |
| *r* + *1* | 12.1 (± 6.11 %) | 22.5 (± 54.9 %) | 17.0 (± 4.08 %) |
| *µ₀* (*cm²*·*V⁻¹*·*s⁻¹)* | 3.95 10⁻⁶ (± 58.2 %) | 7.09·10⁻⁷ (± 8.73 %) | 1.45·10⁻⁵ (± 9.65 %) |
| *γ (cm*^{*1*/*2*}·*V*^{*-1*/}*²)* | 9.82·10⁻⁴ (± 1.23 %) | 4.94·10⁻³ (± 1.27 %) | 4.33·10⁻³ (± 0.78 %) |

### Referenzbeispiel II

Referenzbeispiel II betrifft die Verwendung von Bismut(III)-tris(4-fluorobenzoat), kurz Bi(4-fb)₃, als Metallkomplex für eine Gasphasenabscheidung.

Die Synthese von Bi(4-fb)₃ wurde gemäß Schema 1 durchgeführt. Für Bi(4-fb)₃ sind dabei in Schema 1 die Reste R^{A}, R^{B}, R^{D} und R^{E} Wasserstoffatome und nur R^{C} ein Fluoratom.

Der Erhalt von Bi(4-fb)₃ wurde nach Reinigung mittels Sublimation durch Elementaranalyse bestätigt (gemessen: Kohlenstoff in % 42,4; Wasserstoff in % 2,3; berechnet: Kohlenstoff in % 40,26; Wasserstoff in % 1,92).

Die elektrischen Eigenschaften von mit Bi(4-fb)₃ dotierten organischen Schichten werden in den Figuren 11, 12 und den Tabellen 6, 7 zusammengefasst.

**Tabelle 6: Zusammenfassung der elektrischen Eigenschaften von mit Bi(4-fb)₃ dotiertem 1-TNata.**

| | *(1:8)* | *(1:4)* | *(1:2)* |
|---|---|---|---|
| *Exp. molar ratio* | 1/8.09 (4.54 _{vol.}%) | 1/4.02 (8.74 _{vol.}%) | 1/1.99 (16.20 _{vol.}%) |
| *σ₀ (S·cm⁻¹)* | 2.33·10⁻⁷ (± 1.60 %) | 1.62·10⁻⁷ (± 1.80 %) | 2.50·10⁻⁷ (± 1.21 %) |
| *ρ_{c.;0} (Ω*·*cm*⁻²*)* | no ohmic contact | | 3.80·10⁸ (± 9.84 %) |
| *E_{bi} (kV*·c*m⁻¹)* | 6.38 (± 64.7 %) | 3.13 (± 100 %) | < 0.25 |
| *εᵣ* | 2.75 (± 2.63 %) | 2.52 (± 16.4 %) | 2.91 (± 9.61 %) |
| *r* + *1* | 18.5 (± 8.97 %) | 9.02 (± 2.62 %) | 5.40 (± 2.05 %) |
| *µ₀ (cm²*·*V⁻¹*·*s⁻¹)* | 3.74·10⁻⁵ (± 7.51 %) | 1.19·10⁻⁵ (± 21.5 %) | 3.30·10⁻⁵ (± 15.4 %) |
| *γ (cm*^{*1*/*2*}·*V*^{*-1*/}*²)* | 2.45·10⁻³ (± 1.15 %) | 1.59·10⁻³ (± 0.93 %) | 1.12·10⁻⁴ (± 6.68 %) |

**Tabelle 7: Zusammenfassung der elektrischen Eigenschaften von mit (1:2) Bi(4-fb)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.99 (16.20 _{vol.}%) | 1/2.01 (23.74 _{vol.}%) | 1/1.97 (12.74 _{vol.}%) |
| *σ₀ (S*·*cm⁻¹)* | 2.50·10⁻⁷ (± 1.21 %) | 5.72·10⁻⁷ (± 1.11 %) | 1.99·10⁻⁶ (± 0.81 %) |
| *ρ_{c.;0} (Ω*·*cm⁻²)* | 3.80·10⁸ (± 9.84 %) | no ohmic contact | |
| *E_{bi} (kV*·*cm⁻¹)* | < 0.25 | | 7.75 (± 80.7 %) |
| *εᵣ* | 2.91 (± 9.61 %) | 2.36 (± 7.06 %) | 3.32 (± 7.14 %) |
| *r* + *1* | 5.40 (± 2.05 %) | 7.23 (± 17.06 %) | 13.7 (±2.08%) |
| *µ₀ (cm²*·*V⁻¹*·*s⁻¹)* | 3.30·10⁻⁵ (± 15.4 %) | Trapping | Aging |
| *γ (cm*^{*1*/*2*}·*V*^{*-1*/}*²)* | 1.12·10⁻⁴ (± 6.68 %) | | |

### Referenzbeispiel III

Referenzbeispiel III betrifft die Verwendung von Bismut(III)-tris(3-fluorobenzoat), kurz Bi(3-fb)₃, als Metallkomplex für eine Gasphasenabscheidung.

Die Synthese von Bi(3-fb)₃ wurde gemäß Schema 1 durchgeführt. Für Bi(3-fb)₃ sind dabei in Schema 1 die Reste R^{A}, R^{C}, R^{D} und R^{E} Wasserstoffatome und nur R^{B} ein Fluoratom.

Der Erhalt von Bi(3-fb)₃ wurde nach Reinigung mittels Sublimation durch Elementaranalyse bestätigt (gemessen: Kohlenstoff in % 39,2; Wasserstoff in % 2,3; berechnet: Kohlenstoff in % 40,26; Wasserstoff in % 1,92).

Die elektrischen Eigenschaften von mit Bi(3-fb)₃ dotierten organischen Schichten werden in den Figuren 13, 14 und den Tabellen 8, 9 zusammengefasst.

**Tabelle 8: Zusammenfassung der elektrischen Eigenschaften von mit Bi(3-fb)₃ dotiertem 1-TNata.**

| | *(1:8)* | *(1:4)* | *(1:2)* |
|---|---|---|---|
| *Exp. molar ratio* | 1/8.16 (4.62 _{vol.}%) | 1/3.97 (9.07 ᵥₒₗ.%) | 1/1.94 (16.92 _{vol.}%) |
| *σ₀ (S*·*cm⁻¹)* | 1.73·10⁻⁷ (± 1.51 %) | 1.48·10⁻⁷ (± 0.96 %) | 1.38·10⁻⁷ (± 1.27 %) |
| *ρ_{c.;0} (Ω·cm⁻²)* | no ohmic contact | | 9.07·10⁸ (± 9.56 %) |
| *E_{bi} (kV*·*cm⁻¹)* | 8.38 (± 25.4 %) | 0.63 (± 100 %) | < 0.25 |
| *εᵣ* | 3.57 (± 35.5 %) | 3.22 (± 9.91 %) | 3.64 (± 6.24 %) |
| *r* + *1* | 12.5 (± 3.23 %) | 6.22 (± 1.90 %) | 4.67 (± 2.12 %) |
| *µ₀ (cm²·V⁻¹*·*s⁻¹)* | 9.07 10⁻⁶ (± 40.2 %) | 7.52·10⁻⁶ (± 14.8 %) | 3.37·10⁻⁶ (± 11.6%) |
| *γ (cm*^{*1*/}*²·V*^{*-1*/}*²)* | 1.52 10⁻³ (± 1.04 %) | 1.36·10⁻³ (± 1.19 %) | 1.95·10⁻³ (± 1.13 %) |

**Tabelle 9: Zusammenfassung der elektrischen Eigenschaften von mit (1:2) Bi(3-fb)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.94 (16.92 _{vol.}%) | 1/2.05 (23.85 _{vol.}%) | 1/2.00 (12.86 _{vol.}%) |
| *σ₀ (S*·*cm*⁻*¹)* | 1.38·10⁻⁷ (± 1.27 %) | 6.10·10⁻⁸ (± 0.88 %) | 1.69·10⁻⁶ (± 0. 8 7 %) |
| *ρ_{c.;0} (Ω·cm⁻²)* | 9.07-10⁸ (± 9.56 %) | no ohmic contact | 1.35·10⁴ (± 1.70 %) |
| *E_{bi} (kV*·c*m⁻¹)* | < 0.25 | 21.38 (± 13.5 %) | < 0.25 |
| *εᵣ* | 3.64 (± 6.24 %) | 2.62 (± 7.18 %) | 2.97 (± 15.7 %) |
| *r* + *1* | 4.67 (± 2.12 %) | 17.0 (± 16.9 %) | no TFLC |
| *µ₀ (cm²·V⁻¹·s⁻¹)* | 3.37·10⁻⁶ (± 11.6 %) | Trapping | Aging |
| *γ (cm*^{*1*/}*²·V*^{*1*/}*²)* | 1.95·10⁻³ (± 1.13 %) | | |

### Referenzbeispiel IV

Referenzbeispiel IV betrifft die Verwendung von Bismut(III)-tris(3,5-difluorobenzoat), kurz Bi(3,5-dfb)₃, als Metallkomplex für eine Gasphasenabscheidung.

Die Synthese von Bi(3,5-dfb)₃ wurde gemäß Schema 1 durchgeführt. Für Bi(3,5-dfb)₃ sind dabei in Schema 1 die Reste R^{A}, R^{C} und R^{E} jeweils Wasserstoffatome und die Substituenten R^{B} und R^{D} sind jeweils Fluoratome.

Der Erhalt von Bi(3,5-dfb)₃ wurde nach Reinigung mittels Sublimation durch Elementaranalyse bestätigt (gemessen: Kohlenstoff in % 36,3; Wasserstoff in % 1,4; berechnet: Kohlenstoff in % 37,06; Wasserstoff in % 1,32).

Die elektrischen Eigenschaften von mit Bi(3,5-dfb)₃ dotierten organischen Schichten werden in den Figuren 15, 16 und den Tabellen 10, 11 zusammengefasst.

**Tabelle 10: Zusammenfassung der elektrischen Eigenschaften von mit Bi(3,5-dfb)₃ dotiertem 1-TNata.**

| | *(1:8)* | *(1:4)* | *(1:2)* |
|---|---|---|---|
| *Exp. molar ratio* | 1/8.01 (4.77 _{vol.}%) | 1/3.93 (9.25 _{vol.}%) | 1/1.96 (17.01 _{vol.}%) |
| *σ₀ (S*·*cm⁻¹)* | 4.27·10⁻⁷ (± 1.03 %) | 2.81·10⁻⁷ (± 0.66 %) | 2.97·10⁻⁷ (± 1.31 %) |
| *ρ_{c.;0} (Ω·cm⁻²)* | 2.07·10⁹ (± 8.50 %) | 5.12·10⁷ (± 8.73 %) | 1.43·10⁶ (± 4.98 %) |
| *E_{bi} (kV*·*cm⁻¹)* | < 0.25 | | |
| *εᵣ* | 2.67 (± 7.01 %) | 2.60 (± 9.36 %) | 2.69 (± 10.8 %) |
| *r* + *1* | 6.46 (± 2.28 %) | 3.75 (± 3.36 %) | 1.56 (± 1.90 %) |
| *µ₀ (cm²· V⁻¹*·*s⁻¹)* | 3.14·10⁻⁵ (± 11.9 %) | Ballistic | |
| *γ (cm*^{*1*/*2*}·*V*^{*-1*/}*²)* | 5.09·10⁻⁴ (± 1.16 %) | | |

**Tabelle 11: Zusammenfassung der elektrischen Eigenschaften von mit (1:2) Bi(3,5-dfb)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.96 (17.01 _{vol.}%) | 1/1.99 (24.64 ᵥₒₗ.%) | 1/2.00 (13.05 _{vol.}%) |
| *σ₀ (S*·*cm*⁻*¹)* | 2.97·10⁻⁷ (± 1.31 %) | 7.18·10⁻⁷ (± 1.90 %) | 4.40·10⁻⁶ (± 1.73 %) |
| *ρ_{c.;0} (Ω*·*cm⁻²)* | 1.43·10⁶ (± 4.98 %) | no ohmic contact | 2.91·10⁵ (± 3.76 %) |
| *E_{bi} (kV*·*cm⁻¹)* | < 0.25 | 12.75 (± 35.3 %) | <0.25 |
| *εᵣ* | 2.69 (± 10.8 %) | 2.35 (± 3.84 %) | Too conductive |
| *r* + *1* | 1.56 (± 1.90 %) | 12.9 (± 21.4 %) | no TFLC |
| *µ₀ (cm²*·*V⁻¹*·*s⁻¹)* | Ballistic | Trapping | Aging |
| *γ (cm*^{*1*/}*²·V*^{*-1*/}*²)* | | | |

### Referenzbeispiel V

Referenzbeispiel V betrifft die Verwendung von Bismut(III)-tris(3,4,5-trifluorobenzoat), kurz Bi(3,4,5-tfb)₃, als Metallkomplex für eine Gasphasenabscheidung.

Die Synthese von Bi(3,4,5-tfb)₃ wurde gemäß Schema 1 durchgeführt. Für Bi(3,4,5-tfb)₃ sind dabei in Schema 1 die Reste R^{A} und R^{E} jeweils Wasserstoffatome und die übrigen Substituenten R^{B}, R^{C} und R^{D} sind jeweils Fluoratome.

Der Erhalt von Bi(3,4,5-tfb)₃ wurde nach Reinigung mittels Sublimation durch Elementaranalyse bestätigt (gemessen: Kohlenstoff in % 33,8; Wasserstoff in % 1,2; berechnet: Kohlenstoff in % 34,33; Wasserstoff in % 0,82).

Die elektrischen Eigenschaften von mit Bi(3,4,5-tfb)₃ dotierten organischen Schichten werden in den Figuren 17, 18 und den Tabellen 12, 13 zusammengefasst.

**Tabelle 12: Zusammenfassung der elektrischen Eigenschaften von mit Bi(3,4,5-tfb)₃ dotiertem 1-TNata.**

| | *(1:8)* | *(1:4)* | *(1:2)* |
|---|---|---|---|
| *Exp. molar ratio* | 1/8.15 (5.59 _{vol.}%) | 1/3.92 (10.97 _{vol.}%) | 1/1.96 (19.73 _{vol.}%) |
| *σ₀ (S·cm⁻¹)* | 6.45·10⁻⁷ (± 1.00 %) | 9.73·10⁻⁷ (± 0.88 %) | 1.09·10⁻⁶ (± 0.76%) |
| *ρ_{c.;0} (Ω·cm⁻²)* | 3.24·10⁷ (± 6.25 %) | 1.21·10⁶ (± 5.02 %) | 1.78·10⁵ (± 4.25 %) |
| *E_{bi} (kV*·*cm⁻¹)* | < 0.25 | | |
| *εᵣ* | Too conductive | | |
| *r* + *1* | 4.12 (± 2.46 %) | 2.22 (± 1.78 %) | 1.48 (± 2.46 %) |
| *µ₀ (cm²*·*V⁻¹*·*s⁻¹)* | 6.13·10⁻⁵ (± 25 %)* | Ballistic | |
| *γ (cm*^{*1*/*2*}·*V*^{*-1*/}*²)* | 5.28·10⁻⁴ (± 1.6 %)* | | |

**Tabelle 13: Zusammenfassung der elektrischen Eigenschaften von mit (1:2) Bi(3,4,5-tfb)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.96 (19.73 ᵥₒₗ.%) | 1/2.00 (28.22 _{vol.}%) | 1/2.01 (15.23 _{vol.}%) |
| *σ₀ (S·cm⁻¹)* | 1.09·10⁻⁶ (± 0.76 %) | 1.77·10⁻⁶ (± 1.68 %) | 1.19·10⁻⁵ (± 0.94 %) |
| *ρ_{c.;0} (Ω*·*cm⁻²)* | 1.78·10⁵ (± 4.25 %) | 5.39·10⁷ (± 8.77 %) | 2.06·10⁵ (± 5.68 %) |
| *E_{bi} (kV*·*cm⁻¹)* | < 0.25 | | |
| *εᵣ* | Too conductive | 2.26 (± 12.2 %) | Too conductive |
| *r* + *1* | 1.48 (± 2.46 %) | 3.28 (± 2.22 %) | 3.28 (± 3.72 %) |
| *µ₀ (cm²*·*V⁻¹*·*s⁻¹)* | Ballistic | 9.39·10⁻⁶ (± 19.1 %) | 3.37·10⁻³ (± 23 %)* |
| *γ (cm*^{*1*/*2*}·*V*^{*-1*/}*²)* | | 3.42·10⁻³ (± 3.90 %) | 2.01·10⁻⁴ (± 3.1 %)* |

### Referenzbeispiel VI

Referenzbeispiel VI betrifft die Verwendung von Bismut(III)-tris(perfluorobenzoat), kurz Bi(pfb)₃, als Metallkomplex für eine Gasphasenabscheidung.

Die Synthese von Bi(pfb)₃ wurde gemäß Schema 1 durchgeführt. Für Bi(pfb)₃ sind dabei in Schema 1 alle fünf Reste R^{A} bis R^{E} jeweils Fluoratome.

Der Erhalt von Bi(pfb)₃ wurde nach Reinigung mittels Sublimation durch Elementaranalyse bestätigt (gemessen: Kohlenstoff in % 29,9(6); berechnet: Kohlenstoff in % 29,93). Die elektrischen Eigenschaften von mit Bi(pfb)₃ dotierten organischen Schichten werden in den Figuren 19, 20 und den Tabellen 14, 15 zusammengefasst.

**Tabelle 14: Zusammenfassung der elektrischen Eigenschaften von mit Bi(pfb)₃ dotiertem 1-TNata.**

| | *(1:8)* | *(1:4)* | *(1:2)* |
|---|---|---|---|
| *Exp. molar ratio* | 1/8.08 (5.18 _{vol.}%) | 1/3.98 (9.98 _{vol.}%) | 1/1.97 (18.28 ᵥₒₗ.%) |
| *σ₀ (S*·*cm⁻¹)* | 9.81·10⁻⁷ (± 1.62 %) | 2.65·10⁻⁶ (± 1.73 %) | 6.06·10⁻⁶ (± 1.28 %) |
| *ρ_{c.;0} (Ω*·*cm⁻²)* | 4.27·10⁶ (± 6.35 %) | 9.88·10⁴ (± 3.41 %) | 8.26·10³ (± 2.96 %) |
| *E_{bi} (kV*·*cm⁻¹)* | < 0.25 | | |
| *εᵣ* | 2.92 (± 4.86 %) | Too conductive | |
| *r* + *1* | 3.76 (± 4.99 %) | 2.00 (± 2.34 %) | no TFLC |
| *µ₀ (cm²*·*V⁻¹*·*s⁻¹)* | Ballistic | | |
| *γ (cm*^{*1*/*2*}·*V*^{*-1*/}*²)* | | | |

**Tabelle 15: Zusammenfassung der elektrischen Eigenschaften von mit (1:2) Bi(pfb)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.97 (18.28 _{vol.}%) | 1/2.00 (26.38 _{vol.}%) | 1/1.88 (14.90 _{vol.}%) |
| *σ₀ (S*·*cm⁻¹)* | 6.06·10⁻⁶ (± 1.28 %) | 2.78·10⁻⁶ (± 0.96%) | 9.52·10⁻⁵ (± 1.40 %) |
| *ρ_{c.;0} (Ω·cm⁻²)* | 8.26·10³ (± 2.96 %) | 1.37·10⁸ (± 8.22 %) | 1.92·10⁴ (± 3.24 %) |
| *E_{bi} (kV*·*cm⁻¹)* | < 0.25 | | |
| *εᵣ* | Too conductive | 2.43 (± 7.17 %) | Too conductive |
| *r* + *1* | no TFLC | 4.97 (± 2.34 %) | 2.98 (± 1.77 %) |
| *µ₀ (cm²*·*V⁻¹*·*s⁻¹)* | Ballistic | 4.88·10⁻⁵ (± 10.5 %) | Compliance |
| *γ (cm*^{*1*/*2*}·*V*^{*-1*/}*²)* | | 1.14·10⁻³ (± 0.84 %) | |

### Referenzbeispiel VII

Referenzbeispiel VII betrifft die Verwendung von Bismut(III)-tris(4-perfluorotoluat), kurz Bi(4-pftl)₃, als Metallkomplex für eine Gasphasenabscheidung.

Für Bi(4-pftl)₃ sind dabei in Schema 1 die Reste R^{A}, R^{B}, R^{D} und R^{E} jeweils Fluoratome und R^{C} ist eine CF₃-Gruppe.

Der Erhalt von Bi(4-pftl)₃ wurde nach Reinigung mittels Sublimation durch Elementaranalyse bestätigt (gemessen: Kohlenstoff in % 30,0; berechnet: Kohlenstoff in % 29,03).

Die elektrischen Eigenschaften von mit Bi(4-pftl)₃ dotierten organischen Schichten werden in den Figuren 21, 22 und den Tabellen 16, 17 zusammengefasst.

**Tabelle 16: Zusammenfassung der elektrischen Eigenschaften von mit Bi(4-pftl)₃ dotiertem 1-TNata.**

| | *(1:8)* | *(1:4)* | *(1:2)* |
|---|---|---|---|
| *Exp. molar ratio* | 1/8.10 (5.63 _{vol.}%) | 1/4.02 (10.75 _{vol.}%) | 1/1.97 (19.68 _{vol.}%) |
| *σ₀ (S*·*cm⁻¹)* | 1.07·10⁻⁶ (± 0.81 %) | 2.62·10⁻⁶ (± 0.89 %) | 4.13·10⁻⁶ (± 1.20 %) |
| *ρ_{c.;0} (Ω*·*cm⁻²)* | 9.30·10⁵ (± 3.67 %) | 2.31·10⁵ (± 3.03 %) | 3.83·10⁴ (± 2.85 %) |
| *E_{bi} (kV*·*cm⁻¹)* | < 0.25 | | |
| *εᵣ* | Too conductive | | |
| *r* + *1* | 2.44 (± 29.0 %) | 1.77 (± 2.86 %) | no TFLC |
| *µ₀ (cm²*·*V⁻¹*·*s⁻¹)* | Ballistic | | |
| *γ (cm*^{*1*/*2*}·*V*^{*-1*/}*²)* | | | |

**Tabelle 17: Zusammenfassung der elektrischen Eigenschaften von mit (1:2) Bi(4-pftl)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.97 (19.68 _{vol.}%) | 1/2.04 (27.82 _{vol.}%) | 1/2.03 (15.13 _{vol.}%) |
| *σ₀ (S*·*cm⁻¹)* | 4.13·10⁻⁶ (± 1.20 %) | 3.20·10⁻⁶ (± 0.84 %) | 1.53·10⁻⁴ (± 1.02 %) |
| *ρ_{c.;0} (Ω.cm⁻²)* | 3.83·10⁴ (± 2.85 %) | 3.41·10⁶ (± 3.72 %) | 1.25·10⁴ (± 3.93 %) |
| *E_{bi} (kV*·*cm⁻¹)* | < 0.25 | | |
| *εᵣ* | Too conductive | 2.10 (± 27.4 %) | // |
| *r* + *1* | no TFLC | 1.42 (± 2.07 %) | 2.52 (± 2.27 %) |
| *µ₀ (cm²*·*V⁻¹·s⁻¹)* | Ballistic | | Compliance |
| *γ (cm*^{*1*/*2*}·*V*^{*1*/}*²)* | | | |

Auch weitere herkömmliche Metallkomplexe mit Liganden auf Acetat- und Trifluoracetat-Basis wurden von den Erfindern zum Vergleich mit den im beschriebenen Verfahren verwendeten Komplexen herangezogen:

### Referenzbeispiel VIII

Referenzbeispiel VIII betrifft die Verwendung von Bismut(III)-tris(trifluoracetat), kurz Bi(tfa)₃, als Metallkomplex für eine Gasphasenabscheidung. Die Herstellung ist in der Literatur beschrieben (z.B. Suzuki, H.; Matano, Y. in Organobismut Chemistry, Elsevier 2001).

Die elektrischen Eigenschaften von mit Bi(tfa)₃ dotierten organischen Schichten werden in den Figuren 23, 24 und den Tabellen 18, 19 zusammengefasst.

**Tabelle 18: Zusammenfassung der elektrischen Eigenschaften von mit Bi(tfa)₃ dotiertem 1-TNata.**

| | *(1:8)* | *(1:4)* | *(1:2)* |
|---|---|---|---|
| *Exp. molar ratio* | 1/8.24 (2.37 _{vol.}%) | 1/3.92 (4.85 _{vol.}%) | 1/1.97 (9.22 _{vol.}%) |
| *σ₀ (S*·*cm⁻¹)* | 2.06·10⁻⁶ (± 0.95 %) | 4.47·10⁻⁶ (± 1.23 %) | 7.53·10⁻⁶ (± 1.21 %) |
| *ρ_{c.;0} (Ω*·*cm⁻²)* | 6.82·10⁵ (± 3.31 %) | 2.10·10⁴ (± 2.34 %) | 9.40·10³ (± 2.50 %) |
| *E_{bi} (kV*·*cm⁻¹)* | < 0.25 | | |
| *εᵣ* | Too conductive | | |
| *r* + *1* | 2.45 (± 1.50 %) | no TFLC | no TFLC |
| *µ₀ (cm²*·*V⁻¹·s⁻¹)* | After TFLC, the slope decreases to a limit near 3/2 (ballistic), Just before exponentially increasing (aging) | | |
| *γ (cm*^{*1*/*2*}·*V*^{*-1*/}*²)* | | | |

Mit "After TFLC, the slope decreases to a limit near 3/2 (ballistic)" und "Just before exponentially increasing (aging)" wird auf einen Übergang von einem Ladungstransport-Regime in ein andere verwiesen.

**Tabelle 19: Zusammenfassung der elektrischen Eigenschaften von mit (1:2) Bi(tfa)₃ dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/1.97 (9.22 _{vol.}%) | 1/1.96 (14.21 _{vol.}%) | 1/1.97 (7.05 _{vol.}%) |
| *σ₀ (S*·*cm⁻¹)* | 7.53·10⁻⁶ (± 1.21 %) | 3.97·10⁻⁵ (± 0.94 %) | 2.28·10⁻⁴ (± 0.79 %) |
| *ρ_{c.;0} (Ω*·*cm⁻²)* | 9.40·10³ (± 2.50 %) | 3.80·10⁴ (± 2.13 %) | 1.05·10⁴ (± 3.03 %) |
| *E_{bi} (kV*·*cm⁻¹)* | < 0.25 | | |
| *εᵣ* | Too conductive | | |
| *r* + *1* | no TFLC | 2.50 (± 2.54 %) | 2.58 (± 2.99 %) |
| *µ₀ (cm²*·*V⁻¹*·*s⁻¹)* | Ballistic | Aging | |
| *γ (cm*^{*1*/*2*}·*V*^{*-1*/}*²)* | | | |

Aus den Daten ist ersichtlich, dass Komplexe mit nicht fluorierten Liganden auch dotieren, jedoch deutlich schlechter als Komplexe mit fluorierten Liganden. Auch diese Komplexe sind ebenso wie die anderen Referenzbeispiele nicht für Quellen geeignet, bei welchen Stöße mit zumindest einer Wand der Quelle erfolgen.

### Referenzbeispiel IX

Referenzbeispiel IX betrifft die Verwendung von Bismut(III)-tris(triacetat), kurz Bi(ac)₃, als Metallkomplex für eine Gasphasenabscheidung. Der Komplex ist kommerziell erhältlich.

Die elektrischen Eigenschaften von mit Bi(ac)₃ dotierten organischen Schichten werden in den Figuren 25, 26 und den Tabellen 20, 21 zusammengefasst.

**Tabelle 20: Zusammenfassung der elektrischen Eigenschaften von mit Bi(ac)₃ dotiertem 1-TNata.**

| | *(1:8)* | *(1:4)* | *(1:2)* |
|---|---|---|---|
| *Exp. molar ratio* | 1/8.16 (1.46 _{vol.}%) | 1/4.20 (2.80 _{vol.}%) | 1/2.03 (5.64 _{vol.}%) |
| *σ₀ (S*·*cm⁻¹)* | 4.31·10⁻⁷, (± 1.90 %) | 4.06·10⁻⁷ (± 0.86 %) | 6.13·10⁻⁷ (± 0.81 %) |
| *ρ_{c.;0} (Ω*·*cm⁻²)* | no ohmic contact | 8.41·10⁸ (± 9.79 %) | 5.07· 10⁹ (± 9.63 %) |
| *E_{bi} (kV*·*cm⁻¹)* | 3.13 (± 100 %) | < 0.25 | |
| *εᵣ* | 2.67 (± 37.3 %) | 2.10 (± 10.4 %) | 2.78 (± 6.88 %) |
| *r* + *1* | 9.87 (± 2.98 %) | 5.70 (± 2.73 %) | 7.31 (± 1.24 %) |
| *µ₀ (cm²*·*V⁻¹*·*s⁻¹)* | 2.81·10⁻⁵ (± 42.0 %) | 5.12·10⁻⁵ (± 15.1 %) | 4.46·10⁻⁵ (± 11.8 %) |
| *γ (cm*^{*1*/*2*}·*V*^{*-1*/}*²)* | 1.29·10⁻³ (± 0.80 %) | 3.19·10⁻⁴ (± 1.88 %) | 6.76·10⁻⁴ (± 1.33 %) |

**Tabelle 21: Zusammenfassung der elektrischen Eigenschaften von mit (1:2) Bi(ac)₃-dotierten Matrixmaterialien 1-TNata, α-NPB und spiro-TTB.**

| | *1-TNata* | *α-NPB* | *spiro-TTB* |
|---|---|---|---|
| *Exp. molar ratio* | 1/2.03 (5.64 _{vol.}%) | 1/1.99 (8.99 _{vol.}%) | 1/2.05 (4.23 _{vol.}%) |
| *σ₀ (S*·*cm⁻¹)* | 6.13·10⁻⁷ (± 0.81 %) | 6.10·10⁻¹⁰ (± 16.0 %) | 1.68·10⁻⁷ (± 8.76 %) |
| *ρ_{c.;0} (Ω·cm⁻²)* | 5.07·10⁹ (± 9.63 %) | no ohmic contact | |
| *E_{bi} (kV*·*cm⁻¹)* | < 0.25 | 16.3 (± 10.8 %) | 18.4 (± 12.9 %) |
| *εᵣ* | 2.78 (± 6.88 %) | 2.06 (± 5.58 %) | 2.76 (± 4.44 %) |
| *r* + *1* | 7.31 (± 1.24 %) | 11.9 (± 12.7 %) | 13.0 (± 3.18 %) |
| *µ₀ (cm²*·*V⁻¹*·*s⁻¹)* | 4.46·10⁻⁵ (± 11.8 %) | Trapping | |
| *γ (cm*^{*1*/*2*} ·*V*^{*-1*/}*²)* | 6.76·10⁻⁴ (± 1.33 %) | | |

Die Mehrzahl der genannten Beispiele, Beispiel I, Beispiel II sowie die meisten Referenzbeispiele besitzen ausreichend gute Dotierstoffstärken. Mit diesen Komplexen dotierte Matrixmaterialien zeigen im Falle von Beispiel I und II sowie auch bei einer Vielzahl von Referenzbeispielen hinreichend gute elektrische Leitfähigkeiten.

Im Hinblick auf die Komplexstabilität insbesondere die thermische Stabilität genügen jedoch nur die Metallkomplexe von Vergleichsbeispiel I und Beispiel II den hohen Anforderungen für eine Abscheidung mittels Quellen bei welchen es zu Stößen des Komplexes mit zumindest einer Wand der Quelle kommt. Von den Komplexen der Referenzbeispiele zeigte dagegen kein Komplex eine ausreichende Stabilität um mittels Quellen aus der Gasphase abgeschieden zu werden, bei welchen es zu Stößen mit Wänden der Quelle kommt.

Beispielsweise lassen sich nur die Komplexe von Vergleichsbeispiel I und Beispiel II mittels Linearquellen abscheiden, aber nicht die Komplexe der Referenzbeispiele. Während alle Komplexe stabil genug für eine Abscheidung mittels Punktquellen sind, erfüllen also nur Komplexe mit zumindest einem Substituenten R¹ die hohen Stabilitätsanforderungen.

Die einzelnen Kombinationen der Bestandteile und der Merkmale von den bereits erwähnten Ausführungen sind exemplarisch; der Austausch und die Substitution dieser Lehren mit anderen Lehren, die in dieser Druckschrift enthalten sind mit den zitierten Druckschriften werden ebenfalls ausdrücklich erwogen. Entsprechend ist die obengenannte Beschreibung beispielhaft und nicht als beschränkend anzusehen. Das in den Ansprüchen verwendete Wort "umfassen" schließt nicht andere Bestandteile oder Schritte aus. Der unbestimmte Artikel "ein" schließt nicht die Bedeutung eines Plurals aus. Die bloße Tatsache, dass bestimmte Maße in gegenseitig verschiedenen Ansprüchen rezitiert werden, verdeutlicht nicht, dass eine Kombination von diesen Maßen nicht zum Vorteil benutzt werden kann. Der Umfang der Erfindung ist in den folgenden Ansprüchen definiert und den dazugehörigen Äquivalenten. Diese Patentanmeldung beansprucht die Priorität der deutschen Patentanmeldung 102014114231.4

## Patentansprüche

1. Organisches elektronisches Bauteil umfassend zumindest eine organische elektronische Schicht, wobei die organische elektronische Schicht eine Matrix aufweist, die als Dotand einen Bismutkomplex enthält, wobei der Bismutkomplex mindestens einen an das Bismutatom gebundenen Liganden L enthält, wobei die Liganden L unabhängig voneinander die folgende allgemeine Struktur aufweisen : 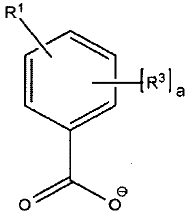 wobei der Substituent R¹ ausgewählt ist aus der Gruppe umfassend verzweigte oder unverzweigte, fluorierte aliphatische Kohlenwasserstoffe mit 1 bis 10 C-Atomen, und wobei der Substituent R³ ausgewählt ist aus der Gruppe umfassend verzweigte oder unverzweigte fluorierte oder nicht fluorierte aliphatische Kohlenwasserstoffe mit 1 bis 10 C-Atomen, Aryl und Heteroaryl, wobei der a gleich 0 oder 1 ist.

2. Organisches elektronisches Bauteil gemäß dem vorherigen Anspruch, wobei L unabhängig ausgewählt ist aus der Gruppe umfassend

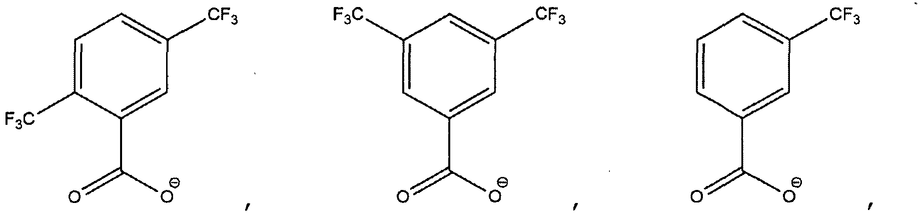

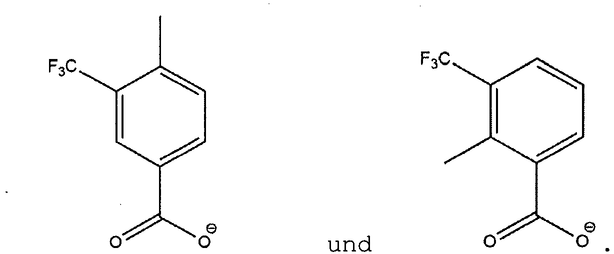

3. Organisches elektronisches Bauteil gemäß einem der vorherigen Ansprüche, wobei der Bismutkomplex der Komplex 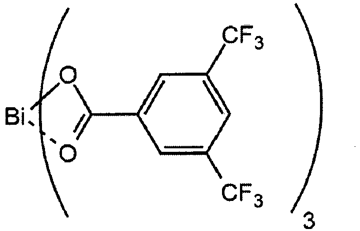 ist.

## Claims

1. Organic electronic component comprising at least one organic electronic layer, wherein the organic electronic layer comprises a matrix which contains as dopant a bismuth complex, wherein the bismuth complex contains at least one ligand L attached to the bismuth atom, wherein the ligands L comprise independently from each other the following general structure: 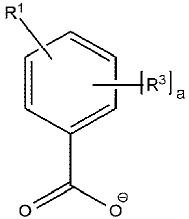 wherein substituent R¹ is selected from the group comprising branched or unbranched, fluorinated aliphatic hydrocarbons with 1 to 10 C atoms, and
wherein substituent R³ is selected from the group comprising branched or unbranched fluorinated or unfluorinated aliphatic hydrocarbons with 1 to 10 C atoms, aryl and heteroaryl, wherein a is equal to 0 or 1.

2. Organic electronic component according to the preceding claim, wherein L is independently selected from the group comprising

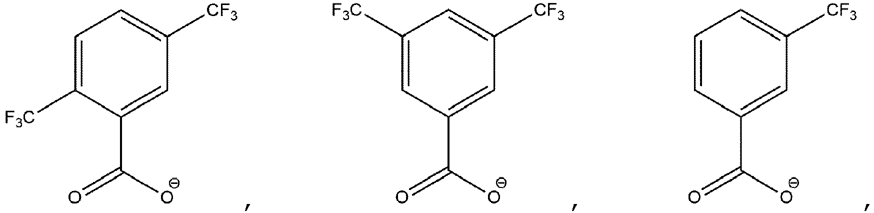

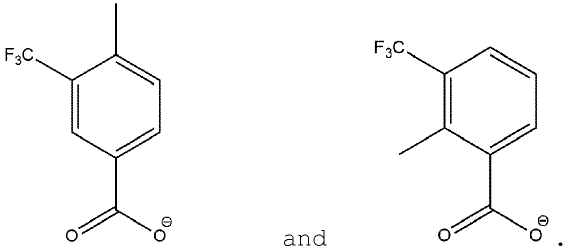

3. Organic electronic component according to one of the preceding claims, wherein the bismuth complex is the complex

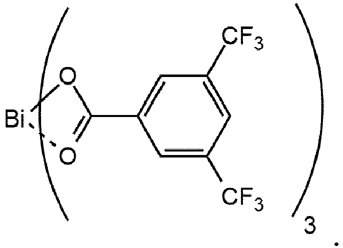

## Revendications

1. Composant électronique organique comprenant au moins une couche électronique organique, dans lequel la couche électronique organique présente une matrice qui, en tant que dopant, contient un complexe de bismuth, le complexe de bismuth contenant au moins un ligand L lié à l'atome de bismuth, les ligands L présentant indépendamment les uns des autres la structure générale suivante : 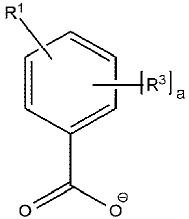
le substituant R¹ est choisi dans le groupe constitué par les hydrocarbures aliphatiques fluorés ramifiés ou non ramifiés comprenant entre 1 et 10 atomes de carbone, et
le substituant R³ est choisi dans le groupe constitué par les hydrocarbures aliphatiques fluorés ou non fluorés et ramifiés ou non ramifiés comprenant entre 1 et 10 atomes de carbone, un aryle et un hétéroaryle, où a est égal à 0 ou 1.

2. Composant électronique organique selon la revendication précédente, dans lequel L est choisi indépendamment dans le groupe constitué par

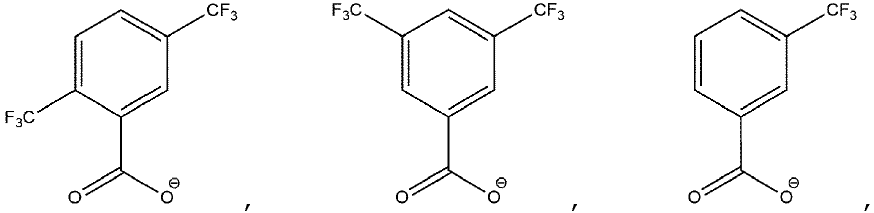

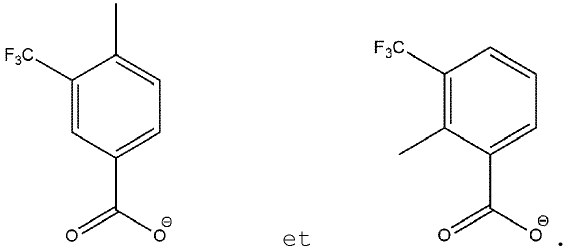

3. Composant électronique organique selon l'une des revendications précédentes, dans lequel le complexe de bismuth est le complexe.
